(19)

**Europäisches Patentamt**
**European Patent Office**
**Office européen des brevets**

(11) **EP 2 130 504 A1**

(12) **EUROPEAN PATENT APPLICATION**
published in accordance with Art. 153(4) EPC

(43) Date of publication:
**09.12.2009 Bulletin 2009/50**

(21) Application number: **07851065.8**

(22) Date of filing: **21.12.2007**

(51) Int Cl.:
**A61B 17/28** (2006.01)       **A61B 1/00** (2006.01)
**A61B 18/12** (2006.01)

(86) International application number:
**PCT/JP2007/074713**

(87) International publication number:
**WO 2008/120426 (09.10.2008 Gazette 2008/41)**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **29.03.2007 JP 2007086482**

(71) Applicant: **Olympus Medical Systems Corporation Tokyo 151-0072 (JP)**

(72) Inventors:
• **NAITO, Kimihiko**
  **Tokyo 192-8512 (JP)**

• **ISHIGURO, Tsutomu**
  **Tokyo 192-8512 (JP)**
• **HASEGAWA, Jun**
  **Tokyo 192-8512 (JP)**
• **NAKAMURA, Toshio**
  **Tokyo 192-8512 (JP)**

(74) Representative: **von Hellfeld, Axel**
**Wuesthoff & Wuesthoff**
**Patent- und Rechtsanwälte**
**Schweigerstrasse 2**
**81541 München (DE)**

(54) **ARTICULATED BENDING MECHANISM AND ARTICULATED MEDICAL DEVICE WITH ARTICULATED BENDING MECHANISM**

(57)     In a first bending piece (51), a second bending piece (52), and a third bending piece (53) disposed sequentially from an extreme end side, manipulation wires (56a, 56b) for rotating the first bending piece (51), manipulation wires (57a, 57b) for rotating the second bending piece (52), and manipulation wires (58a, 58b) for rotating the third bending piece (53), the manipulation wires (56a, 56b) are disposed inwards of the manipulation wires (57a, 57b), and the manipulation wires (56a, 56b) and the manipulation wires (57a, 57b) are disposed inwards of the manipulation wires (58a, 58b).

FIG. 5B

## EP 2 130 504 A1

**Description**

Technical Field

The present invention relates to a multijointed bending mechanism in which a plurality of bending pieces can be independently manipulated by manipulation wires, and to a multijointed medical equipment having such multijointed bending mechanism.

Background Art

In general, an insertion portion of a piece of medical equipment such as an endoscope is provided with a bending portion. Bending pieces are rotatably coupled with each other in the bending portion. A manipulation wire is connected only to a bending piece at the most extreme end of the bending portion. The bending portion is bend in its entirety by pushing and pulling the manipulation wire. More specifically, since the respective bending pieces cannot be independently rotated, it is difficult for the bending pieces to take a predetermined bending state.

To cope with the above problem, in Patent Document 1, repellent force application means is disposed in the base end portion of a bending portion. When the bending portion is bend by a manipulation wire, the repellent force application means begins to bend the bending portion preferentially from the extreme end portion thereof. Further, in Patent Document 2, balloons are interposed between bending pieces, respectively. Rotation intervals between bending pieces are adjusted by the expansion and contraction of the balloons. With this configuration, when a bending portion is bend, the radius of bendature of the bending portion can be variably adjusted.

Patent Document 1: Jpn. Pat. Appln. KOKAI Publication No. 2003-126024
Patent Document 2: Jpn. Pat. Appln. KOKAI Publication No. 06-105797

Disclosure of Invention

When the bending state of the bending portion is regulated by the repellent force application means as disclosed in Patent Document 1 or by the balloons as disclosed in Patent Document 2, only a part of the bending portion is preferentially bent. Accordingly, since it is impossible to selectively rotate an arbitrary bending piece, there is a possibility that a desired bending state cannot be attained.

When manipulation wires are disposed in respective bending pieces to increase the degree of freedom of the bending state, the number of the manipulation wires is increased. Accordingly, the number of the disposed manipulation wires is increased toward the extreme end side of the bending portion. As a result, a problem arises in that the degree of freedom of an operation is lowered on the extreme end side of the bending portion.

Further, there is a possibility that the increase in the number of the manipulation wires makes it difficult for the manipulation wires to smoothly execute a bending manipulation. Further, the diameter of an equipment is increased in its entirety. This is particularly disadvantageous when it is desired to reduce the diameter of a medical equipment.

Accordingly, the present invention provides a multijointed bending mechanism, in which only an arbitrary bending pieces can be independently rotated, a bending manipulation can be smoothly executed by manipulation wires, the manipulation wires are disposed compactly without being entangled with each other, and the diameter of a bending portion can be reduced, and multijointed medical equipment having the multijointed bending mechanism.

According to one aspect of the present invention, there is provided a multijointed bending mechanism comprising: a first bending piece; a second bending piece connected to the first bending piece so as to be rotatable around a first rotation shaft; a third bending piece connected to the second bending piece so as to be rotatable around a second rotation shaft; at least two first wires connected to the first bending piece to rotate the first bending piece; and at least two second wires connected to the second bending piece to rotate the second bending piece, wherein the first wires are disposed inwards of the second wires with respect to a vertical direction of the first and second rotation shafts.

According to one aspect of the present invention, there is provided a multijointed bending mechanism comprising: a first bending piece; a second bending piece connected to the first bending piece so as to be rotatable around a first rotation shaft; a third bending piece connected to the second bending piece so as to be rotatable around a second rotation shaft; at least two first wires connected to the first bending piece to rotate the first bending piece; and at least two third wires connected to the third bending piece to rotate the third bending piece, wherein the first wires are disposed inwards of the third wires with respect to a vertical direction of the first and second rotation shafts.

An aspect of the present invention provides a multijointed medical equipment having the multijointed bending mechanism.

Brief Description of Drawings

FIG. 1 is a perspective view schematically showing an endoscope apparatus included in an endoscope system according to an embodiment of the present invention.
FIG. 2 is a perspective view schematically showing an endoscope and a surgical instrument in the endoscope system according to the embodiment.
FIG. 3 is a perspective view schematically showing the surgical instrument according to the embodiment.
FIG. 4 is a perspective view showing an extreme end portion and a bending portion in an insertion portion of the surgical instrument according to the embodiment.
FIG. 5A is a horizontal longitudinal sectional view of the bending portion along line A-A in FIG. 4 in the long axis direction of the insertion portion, as viewed from above.
FIG. 5B is a vertical longitudinal sectional view of the bending portion along line B-B in FIG. 4 in the long axis direction of the insertion portion, as viewed from the left side thereof.
FIG. 6A is a lateral sectional view along a line A-A in FIG. 5B and a view showing the disposition of manipulation wires and guide sheaths.
FIG. 6B is a lateral sectional view along a line B-B in FIG. 5B and a view showing the disposition of the manipulation wires and the guide sheaths.
FIG. 6C is a lateral sectional view along a line C1-C1 in FIG. 5B and a view showing the disposition of the manipulation wires and the guide sheaths.
FIG. 6D is a lateral sectional view along a line C2-C2 in FIG. 5B and a view showing the disposition of the manipulation wires and the guide sheaths.
FIG. 6E is a lateral sectional view along a line D-D in FIG. 5B and a view showing the disposition of the manipulation wires and the guide sheaths.
FIG. 7A shows the angular relationship under which bending pieces rotate and is a longitudinal sectional view of the bending portion from above.
FIG. 7B shows the angular relationship under which the bending pieces rotate and is a longitudinal sectional view of the bending portion as viewed from above.
FIG. 8A is an explanatory view of a multijointed structure in the bending portion of a surgical instrument.
FIG. 8B is an explanatory view of a multijointed structure in the bending portion of the surgical instrument.
FIG. 9 is an explanatory view of a multijointed structure in a joystick.
FIG. 10A shows a modification of the disposition of wire guides and is a lateral sectional view along the line A-A in FIG. 5B when the wire guides are viewed from a front surface thereof.
FIG. 10B shows a modification of the disposition of the wire guides and is a lateral sectional view along the line B-B in FIG. 5B when the wire guides are viewed from the front surface thereof.
FIG. 10C shows a modification of the disposition of the wire guides and is a lateral sectional view along the line C1-C1 in FIG. 5B when the wire guides are viewed from the front surface thereof.
FIG. 10D shows a modification of the disposition of the wire guides and is a lateral sectional view along the line C2-C2 in FIG. 5B from the front surface thereof.
FIG. 10E shows a modification of the disposition of the wire guides and is a lateral sectional view along the line D-D in FIG. 5B when the wire guides are viewed from the front surface thereof.
FIG. 11A shows a modification of the disposition of the wire guides and is a lateral sectional view along the line A-A in FIG. 5B when the wire guides are viewed from the front surface thereof.
FIG. 11B shows a modification of the disposition of the wire guides and is a lateral sectional view along the line B-B in FIG. 5B when the wire guides are viewed from the front surface thereof.
FIG. 11C shows a modification of the disposition of the wire guides and is a lateral sectional view along the line C2-C2 in FIG. 5B when the wire guides are viewed from the front surface thereof.
FIG. 11D shows a modification of the disposition of the wire guides and is a lateral sectional view along the line D-D in FIG. 5B when the wire guides are viewed from the front surface thereof.
FIG. 12 is an explanatory view of a multijointed structure in a bending portion of a surgical instrument in another embodiment of the present invention.
FIG. 13 is an explanatory view of a multijointed structure of a joystick in the embodiment.

Best Mode for Carrying Out the Invention

A multijointed surgical instrument (for example, multijointed medical equipment) having a multijointed bending mechanism according to an embodiment of the present invention and an endoscope system having such multijointed surgical instrument will be explained below with reference to the drawings.

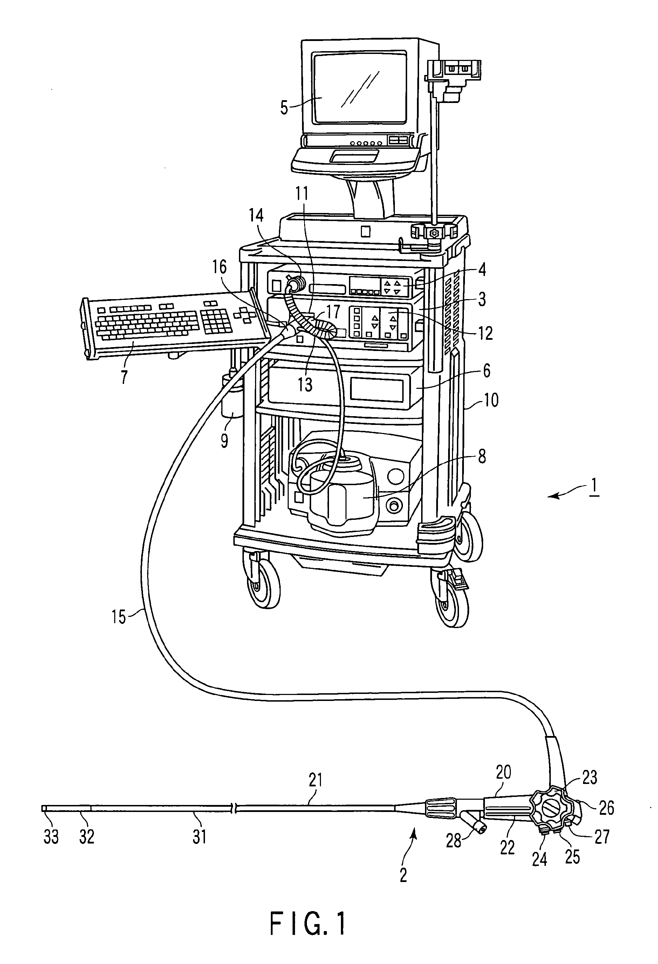

FIG. 1 is a perspective view schematically showing an endoscope apparatus 1 included in the endoscope system. The endoscope apparatus 1 is composed of an electronic endoscope (endoscope main body) 2 and a peripheral device (device main body) of the endoscope 2.

The peripheral device includes a light source unit 3 for creating endoscope illumination light, an image processing unit 4 for subjecting an image picked up by an image pickup portion (not shown) in the endoscope 2 to various types of image processing, an image display unit (for example, monitor) 5 for displaying an image, image data (the image processed by the image processing unit 4), a state of the device, an instruction of an operator, and the like, a controller 6 for overall control of the endoscope system and executing an arithmetic operation and the like, an input unit 7 having a keyboard and the like, a waste fluid tank 8 with a suction pump, a water feed tank 9, and the like. The peripheral device is mounted on a trolley 10.

The light source unit 3 has a connection port 11 connected to a connector unit 16 and a display 12 for displaying an operating state of the light source unit 3 on the front surface thereof.

The image processing unit 4 has a connector receiver 14 connected to a connection cable 13 on a front surface thereof. A connecting unit 17 with a cap is disposed in the base end of the connection cable 13. Further, the connector unit 16 is disposed in the extreme end of a universal cord 15 of the endoscope 2. An electrical connection portion of the connector unit 16 is detachably connected to the connecting unit 17 with the cap.

An image pickup signal obtained in the image pickup portion is sent to the image processing unit 4 through the connection cable 13 and converted to a video signal. The video signal is displayed on the image display unit 5 as an image picked up by the endoscope 2.

Although the endoscope 2 is an electronic endoscope for picking up an endoscope image by an image pick-up portion (not shown image pick-up device) disposed in the extreme end of a later-described insertion portion 21, it may be, for example, a fiber endoscope using an image guide fiber. When the fiber endoscope is used, an optical image guided by the image guide fiber is picked up by a TV camera or the like.

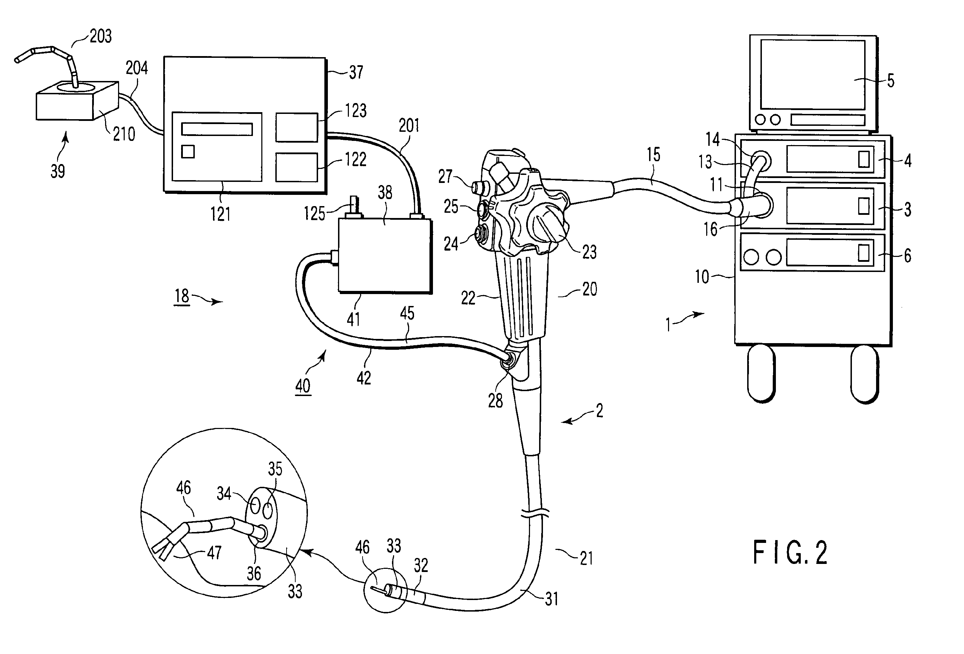

As shown in FIGS. 1 and 2, the endoscope 2 has a manipulation portion 20 and the insertion portion 21 as a base member.

The universal cord 15 is connected to the manipulation portion 20. A grip portion 22 is disposed in the manipulation portion 20. The manipulation portion 20 is provided with various types of function manipulation members, such as an angle manipulation knob 23, an air/water feed manipulation button 24, a suction manipulation button 25, a gas supply manipulation button 26, and switches 27. The function manipulation members are disposed in portions nearer to a proximal end side than the position of the grip portion 22. Further, an insertion port 28 of an insertion channel, into which a later-described surgical instrument 40 and the like are inserted, is disposed in a portion which is positioned nearer to an extreme end side than the position of the grip portion 22.

As shown in FIGS. 1 and 2, the insertion portion 21 is composed of a flexible tube (soft portion) 31 positioned to the proximal end side, a bending portion 32 connecting to the extreme end of the flexible tube 31, and an extreme end portion 33 connected to the extreme end of the bending portion 32. The flexible tube 31 has elasticity and flexibility and is bent by an external force. The bending portion 32 is forcibly bend by manipulating the angle manipulation knob 23. The position and the direction of the extreme end portion 33 are changed by bending the bending portion 32 so that a desired observation target (affected area and the like) is captured in an observation field of view (or in an image pickup field of view).

As shown in FIG. 2, an observation window 34, an illumination window 35, and a channel port 36 are disposed in the extreme end surface portion of the extreme end portion 33.

An image pickup unit, which includes an optical system composed of an objective lens (not shown) and the like and an image pick-up device such as a CCD, is disposed inside the observation window 34. The image pick-up unit picks up an affected area and the like in a body cavity. An image pick-up signal obtained by the image pick-up unit is sent to the image processing unit 4 through the connection cable 13 as described above.

The channel port 36 communicates with the insertion port 28 through an insertion channel (not shown) formed in the insertion portion 21. The insertion channel is used as a path through which an insertion portion 42 of a multijointed surgical instrument 40 for an endoscope is inserted.

Although it is assumed in the embodiment that one surgical instrument 40 is inserted into one insertion channel, a plurality of surgical instruments 40 may be inserted into the one insertion channel. Further, it is also possible to provide a plurality of the insertion channels and to insert each of the surgical instruments 40 into each of the insertion channels.

Next, a surgical instrument extreme end movement controller 18 will be explained with reference to FIGS. 2, 3, 4, 5A and 5B. As shown in FIG. 2, the surgical instrument extreme end movement controller 18 includes a surgical instrument controller 37, a surgical instrument drive unit (motor unit) 38, a bending manipulation unit (manipulation input unit) 39, and the surgical instrument 40.

The surgical instrument 40 includes a manipulation unit 41 which can be gripped by an operator and the insertion portion 42 coupled with the manipulation unit 41.

The surgical instrument drive unit 38 is assembled to the manipulation unit 41.

As shown in FIG. 2, the insertion portion 42 is inserted into a body cavity through the insertion channel. The insertion portion 42 is composed of a flexible tube (soft portion) 45 which is positioned on the proximal end (base end) side, a bending portion 46 connected to the extreme end of the flexible tube 45, and an extreme end portion 47 connected to the extreme end of the bending portion 46.

The flexible tube 45 has elasticity and flexibility and is bent by an external force.

The bending portion 46 is bent by the manipulation unit 41.

The extreme end portion 47 is provided with a grip forceps 48 as a surgical instrument for operating on an affected area and the like.

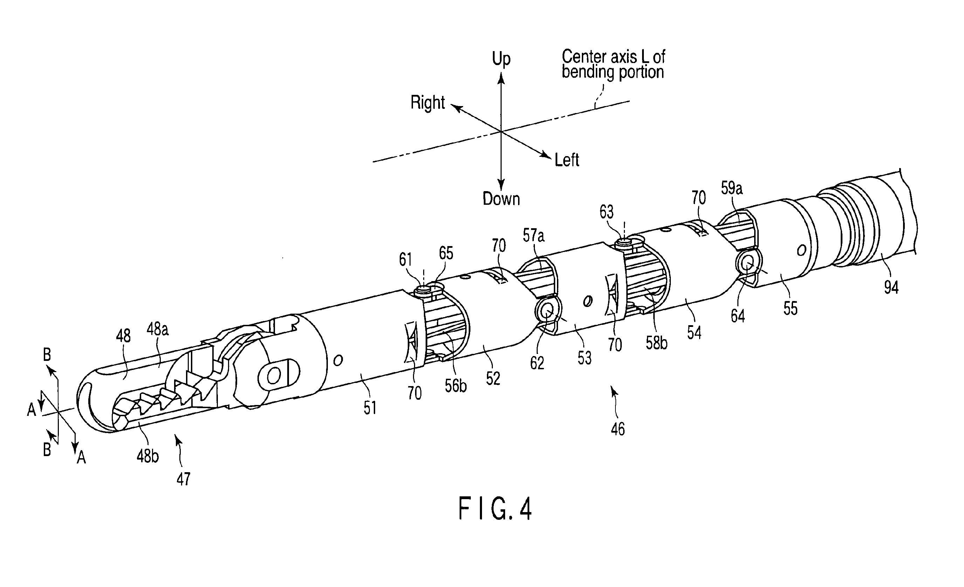

As shown in FIG. 4, the grip forceps 48 includes grip members 48a, 48b which are opened and closed up and down. The grip members 48a, 48b are opened and closed in up and down directions by a manipulation wire 93 inserted into the insertion portion 42. The extreme end portion 47 may be provided with a surgical instrument such as a high-frequency knife or a high-frequency solidifier in addition to the grip forceps 48.

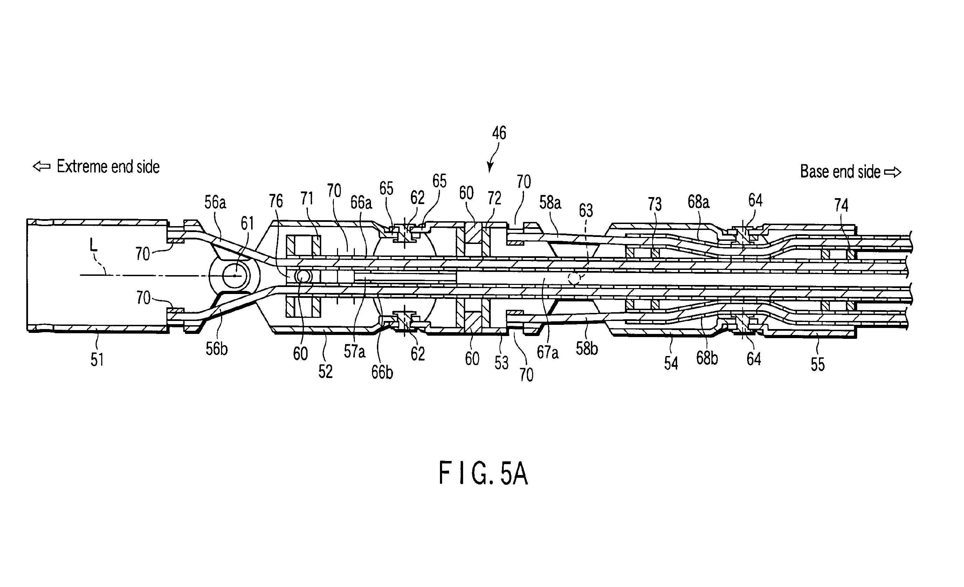

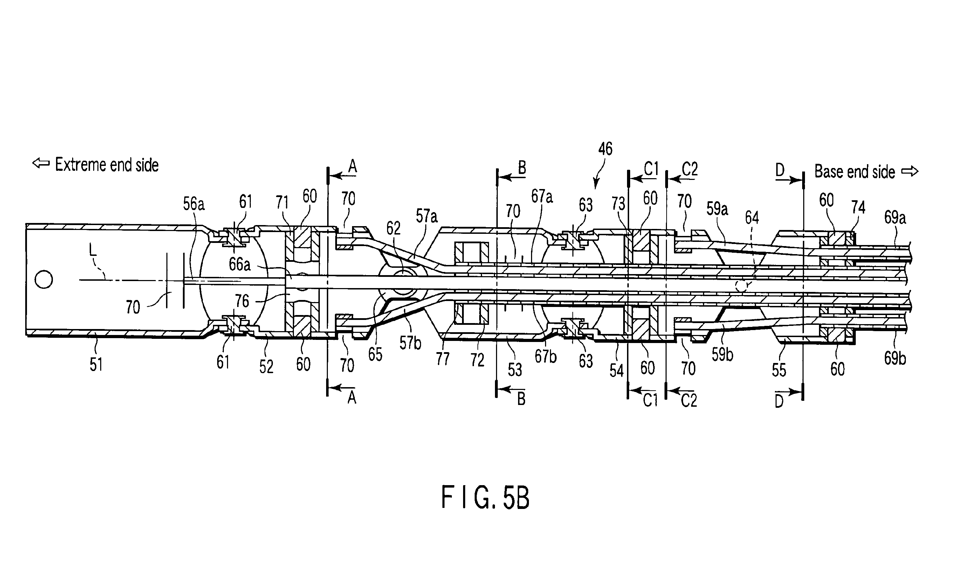

As shown in FIGS. 4, 5A and 5B, the bending portion 46 includes the multijointed bending mechanism. The multijointed mechanism is constructed by coupling bending pieces 51, 52, 53, 54, 55. FIG. 4 is a perspective view showing the extreme end portion 47 and the bending portion 46. FIG. 5A is a horizontal longitudinal sectional view of the bending portion 46 along line A-A in FIG. 4 in the long axis direction of the insertion portion 42 as viewed from above. FIG. 5B is a vertical longitudinal sectional view of the bending portion 46 along line B-B in FIG. 4 in the long axis direction of the insertion portion 42 as viewed from the left side thereof. The up, down, right, and left directions of the bending portion 46 are as shown by indexes of FIG. 4.

The bending pieces 51, 52, 53, 54, 55 are formed of an annular member. As shown in FIG. 4, the bending pieces 51, 52, 53, 54, 55 are disposed by being coaxially arranged in a line in the long axis direction of the insertion portion 42. The bending pieces 51, 52, 53, 54, 55 are sequentially called a first bending piece 51, a second bending piece 52, a third bending piece 53, a fourth bending piece 54, and a fifth bending piece 55 from the extreme end side thereof.

The first and second bending pieces 51, 52 are rotatably connected to each other around a first rotation shaft 61 and rotatably coupled with each other by the first rotation shaft 61. The axial direction of the first rotation shaft 61 is orthogonal to the long axis direction of the insertion portion 42 and the first rotation shaft 61 is disposed in a direction along the up and down directions shown in FIG. 4. Accordingly, the first and second bending pieces 51, 52 can be relatively rotated in right and left directions when viewed from the proximal end (base end) side in FIG. 4.

The second and third bending pieces 52, 53 are rotatably connected to each other around a second rotation shaft 62 and rotatably coupled with each other by the second rotation shaft 62. The axial direction of the second rotation shaft 62 is orthogonal to the long axis direction of the insertion portion 42 and the second rotation shaft 62 is disposed in a direction along the right and left directions shown in FIG. 4. Accordingly, the second and third bending pieces 52, 53 can be relatively rotated in the up and down directions when viewed from the proximal end (base end) side in FIG. 4.

The third and fourth bending pieces 53, 54 are rotatably connected to each other around a third rotation shaft 63 and rotatably coupled with each other by the third rotation shaft 63. The axial direction of the third rotation shaft 63 is orthogonal to the long axis direction of the insertion portion 42 and the third rotation shaft 63 is disposed in the direction along the up and down directions shown in FIG. 4. Accordingly, the third and fourth bending pieces 53, 54 can be relatively rotated in the right and left directions when viewed from the proximal end (base end) side in FIG. 4.

The fourth and fifth bending pieces 54, 55 are rotatably connected to each other around a fourth rotation shaft 64 and rotatably coupled with each other by the fourth rotation shaft 64. The axial direction of the fourth rotation shaft 64 is orthogonal to the long axis direction of the insertion portion 42 and the fourth rotation shaft 64 is disposed in a direction along the right and left directions shown in FIG. 4. Accordingly, the fourth and fifth bending pieces 54, 55 can be relatively rotated in the up and down directions when viewed from the proximal end (base end) side in FIG. 4.

That is, the first rotation shaft 61 constitutes a joint for relatively rotating the first and second bending pieces 51, 52 in the right and left directions. The second rotation shaft 62 constitutes a joint for relatively rotating the second and third bending pieces 52, 53 in the up and down directions. The third rotation shaft 63 constitutes a joint for relatively rotating the third and fourth bending pieces 53, 54 in the right and left directions. Further, the fourth rotation shaft 64 constitutes a joint for relatively rotating the fourth and fifth bending pieces 54, 55 in the up and down directions.

In the embodiment, the axial directions of the first, second, third, and fourth rotation shafts 61, 62, 63, 64 are alternately offset by 90°. That is, the bending pieces 51, 52 and the bending pieces 53, 54 are rotated in the right and left directions. The bending pieces 52, 53 and the bending pieces 54, 55 are rotated in the up and down directions. Further, the axial directions of the rotation shafts 61, 62, 63, 64 are orthogonal to the center axis (long axis) L of the bending portion 46 (refer to FIGS. 4, 5A and 5B). The center axis L agrees with the long axis of the insertion portion 42.

As shown in FIGS. 5A and 5B, the bending pieces 51, 52, 53, 54, 55 have tongue-piece-shaped coupling portions 65 projecting from the end edges thereof. When the coupling portions 65 are overlapped with each other, the rotation shafts 61, 62, 63, 64 pass through the overlapping portions. That is, the rotation shafts 61, 62, 63, 64 are rivet-like shaft members.

The multijointed bending mechanism arranged as described above is covered with a flexible casing (not shown). The bending portion 46 is constructed by this configuration.

A first set of a pair of non-expandable manipulation wires 56 (56a, 56b) connected to the first bending piece 51, a second set of a pair of non-expandable manipulation wires 57 (57a, 57b) connected to the second bending piece 52, a third set of a pair of non-expandable manipulation wires 58 (58a, 58b) connected to the third bending piece 53, and a fourth set of a pair of non-expandable manipulation wires 59 (59a, 59b) connected to the fourth bending piece 54 are inserted into the insertion portion 42.

As shown in FIG. 5A, the manipulation wires 56a, 56b are laterally symmetrically disposed in the bending portion 46 with respect to the center axis L. The extreme ends of the manipulation wires 56a, 56b extend to the region in the first bending piece 51 and are connected to the first bending piece 51.

The direction of the center axis of the first bending piece 51 approximately agrees with the direction of the center axis L. On one plane which passes through both the direction of the center axis of the first bending piece 51 and the axial direction of the first rotation shaft 61, the right half portion of the first bending piece 51 is called a right portion, and the left half portion of the first bending piece 51 is called a left portion.

The extreme end of the manipulation wire 56a described above is connected to the right portion of the first bending piece 51. Further, the extreme end of the manipulation wire 56b is connected to the left portion of the first bending piece 51. When the manipulation wire 56a is pulled to the base end (proximal end) side shown in FIG. 5A, the first bending piece 51 is rotated rightward around the first rotation shaft 61. Further, when the manipulation wire 56b is pulled to the base end side, the first bending piece 51 is rotated leftward around the first rotation shaft 61. As described above, the manipulation wires 56 rotate the first bending piece 51.

As shown in FIG. 5B, the manipulation wires 57a, 57b are vertically symmetrically disposed in the bending portion 46 with respect to the center axis L. The extreme ends of the manipulation wires 57a, 57b extend to the region in the second bending piece 52 and are connected to the second bending piece 52.

The direction of the center axis of the second bending piece 52 approximately agrees with the direction of the center axis L. On one plane which passes through both the direction of the center axis of the second bending piece 52 and the axial direction of the second rotation shaft 62, the upper half portion of the second bending piece 52 is called an upper portion, and the lower half portion of the second bending piece 52 is called a lower portion.

The extreme end of the manipulation wire 57a described above is connected to the upper portion of the second bending piece 52. Further, the extreme end of the manipulation wire 57b is connected to the lower portion of the second bending piece 52. When the manipulation wire 57a is pulled to the base end (proximal end) side shown in FIG. 5B, the second bending piece 52 is rotated upward around the second rotation shaft 62. Further, when the manipulation wire 57b is pulled to the base end side shown in FIG. 5B, the second bending piece 52 is rotated downward around the second rotation shaft 62. As described above, the manipulation wires 57 rotate the second bending piece 52.

As shown in FIG. 5A, the manipulation wires 58a, 58b are laterally symmetrically disposed in the bending portion 46 with respect to the center axis L. The extreme ends of the manipulation wires 58a, 58b extend to the region in the third bending piece 53 and are connected to the third bending piece 53.

The direction of the center axis of the third bending piece 53 approximately agrees with the direction of the center axis L. On one plane which passes through both the direction of the center axis of the third bending piece 53 and the axial direction of the third rotation shaft 63, the right half portion of the third bending piece 53 is called a right portion, and the left half portion of the third bending piece 53 is called a left portion.

The extreme end of the manipulation wire 58a described above is connected to the right portion of the third bending piece 53. Further, the extreme end of the manipulation wire 58b is connected to the left portion of the third bending piece 53. When the manipulation wire 58a is pulled to the base end (proximal end) side shown in FIG. 5A, the third bending piece 53 is rotated rightward around the third rotation shaft 63. Further, when the manipulation wire 58b is pulled to the base end side shown in FIG. 5A, the third bending piece 53 is rotated leftward around the third rotation shaft 63. As described above, the manipulation wires 58 rotate the third bending piece 53.

As shown in FIG. 5B, the manipulation wires 59a, 59b are vertically symmetrically disposed in the bending portion 46 with respect to the center axis L. The extreme ends of the manipulation wires 59a, 59b extend to the region in the fourth bending piece 54 and are connected to the fourth bending piece 54.

The direction of the center axis of the fourth bending piece 54 approximately agrees with the direction of the center axis L. On one plane which passes through both the direction of the center axis of the fourth bending piece 54 and the axial direction of the fourth rotation shaft 64, the upper half portion of the fourth bending piece 54 is called an upper portion, and the lower half portion of the fourth bending piece 54 is called a lower portion.

The extreme end of the manipulation wire 59a described above is connected to the upper portion of the fourth bending piece 54. Further, the extreme end of the manipulation wire 59b is connected to the lower portion of the fourth bending piece 54. When the manipulation wire 59a is pulled to the base end (proximal end) side shown in FIG. 5B, the fourth bending piece 54 is rotated upward around the fourth rotation shaft 64. Further, when the manipulation wire 59b is pulled to the base end side shown in FIG. 5B, the fourth bending piece 54 is rotated downward around the fourth rotation shaft 64. As described above, the manipulation wires 59 rotate the fourth bending piece 54.

As described above, the pairs of the manipulation wires 56, 57, 58, 59, which individually correspond to each other, are connected to the bending pieces 51, 52, 53, 54. When the pairs of the manipulation wires 56, 57, 58, 59 are appropriately selected and pushed and pulled in the bending portion 46, the bending pieces 51, 52, 53, 54 are independently rotated.

Various methods can be employed to connect the extreme ends of the manipulation wires 56, 57, 58, 59 to the bending pieces 51, 52, 53, 54. The connection is made as described below in the embodiment.

As shown in FIG. 5A, in the base end portion of the first bending piece 51, cut and raised pieces 70, which project inside of the first bending piece 51, are formed in the right portion and the left portion of the first bending piece 51. The extreme end of the manipulation wire 56a is inserted into the cut and raised piece 70 in the right portion, and fixed to the cut and raised piece 70 by brazing. Further, the extreme end of the manipulation wire 56b is inserted into the cut and raised piece 70 in the left portion, and fixed to the cut and raised piece 70 by brazing.

As shown in FIG. 5B, in the base end portion of the second bending piece 52, cut and raised pieces 70, which project inside of the second bending piece 52, are formed in the upper portion and the lower portion of the second bending piece 52. The extreme end of the manipulation wire 57a is inserted into the cut and raised piece 70 in the upper portion, and fixed to the cut and raised piece 70 by brazing. Further, the extreme end of the manipulation wire 57b is inserted into the cut and raised piece 70 in the lower portion, and fixed to the cut and raised piece 70 by brazing.

As shown in FIG. 5A, in the periphery of the base end portion of the third bending piece 53, cut and raised pieces 70, which project inside of the third bending piece 53, are formed in the right portion and the left portion of the third bending piece 53. The extreme end of the manipulation wire 58a is inserted into the cut and raised piece 70 in the right portion, and fixed to the cut and raised piece 70 by brazing. Further, the extreme end of the manipulation wire 58b is inserted into the cut and raised piece 70 in the left portion, and fixed to the cut and raised piece 70 by brazing.

As shown in FIG. 5B, in the base end portion of the fourth bending piece 54, cut and raised pieces 70, which project inside of the fourth bending piece 54, are formed in the upper portion and the lower portion of the fourth bending piece 54. The extreme end of the manipulation wire 59a is inserted into the cut and raised piece 70 in the upper portion, and fixed to the cut and raised piece 70 by brazing. Further, the extreme end of the manipulation wire 59b is inserted into the cut and raised piece 70 in the lower portion, and fixed to the cut and raised piece 70 by brazing.

The manipulation wires 56 are inserted into a guide sheath 66, the manipulation wires 57 are inserted into a guide sheath 67, the manipulation wires 58 are inserted into a guide sheath 68, and the manipulation wires 59 are inserted into a guide sheath 69, and they are individually guided up to the manipulation unit 41. The guide sheaths 66, 67, 68, 69 have flexibility and are formed of a sheath-like elastic member having elasticity; for example, an intimately wound coil, a resin tube, and the like. Inner holes of the guide sheaths 66, 67, 68, 69 act as guide members for guiding the direction of travel of the manipulation wires 56, 57, 58, 59.

The extreme end of each guide sheath is not connected to a bending piece to which the manipulation wire to be guided by the guide sheath itself is connected but connected to a bending piece disposed nearer to the base end side than the above bending piece. For example, the extreme ends of guide sheaths 66a, 66b are connected to the second bending piece 52. The extreme ends of guide sheaths 67a, 67b are connected to the third bending piece 53. Further, the extreme ends of guide sheaths 68a, 68b are connected to the fourth bending piece 54.

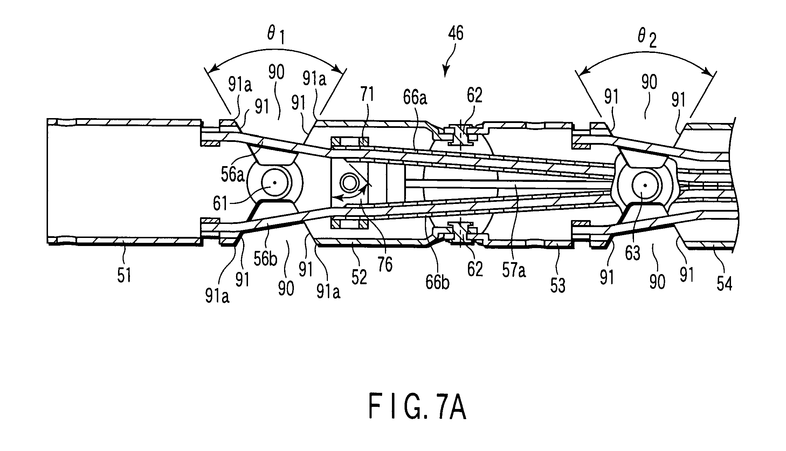

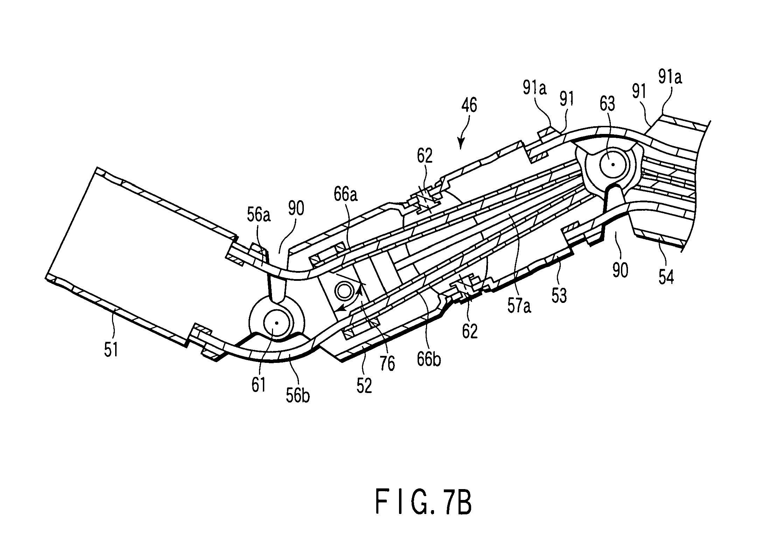

Note that base ends of the guide sheaths may be connected to the base end portion of the bending portion 46 (the extreme end of the flexible tube 45). Further, as shown in FIGS. 7A and 7B, the most extreme end faces of the guide sheaths 66a, 66b may have slant surfaces whose sides near the center of the bending portion 46 retreat to the base end side than the side thereof near the outer periphery of the bending portion 46. As described above, the guide sheaths 66a, 66b may be arranged so that they avoid interference with contained members.

A bending piece, to which the extreme end of a guide sheath is connected, is not a bending piece to which a manipulation wire to be guided by the guide sheath is connected but a bending piece disposed nearer to the base end side than the bending piece to which the manipulation wire is connected. Accordingly, an extreme end of a manipulation wire projecting from the extreme end of a guide sheath is connected to the cut and raised piece 70 disposed in a bending piece disposed in the extreme end of a bending piece to which the extreme end of the guide sheath is connected. That is, the manipulation wires are guided up to the bending pieces which are disposed nearer to the base end side than the bending pieces to which they are connected by being caused to pass in the guide sheaths. Accordingly, a manipulation wire guided by a guide sheath does not directly contact the contained members such as other manipulation wires or guide sheaths so as to avoid interference therewith.

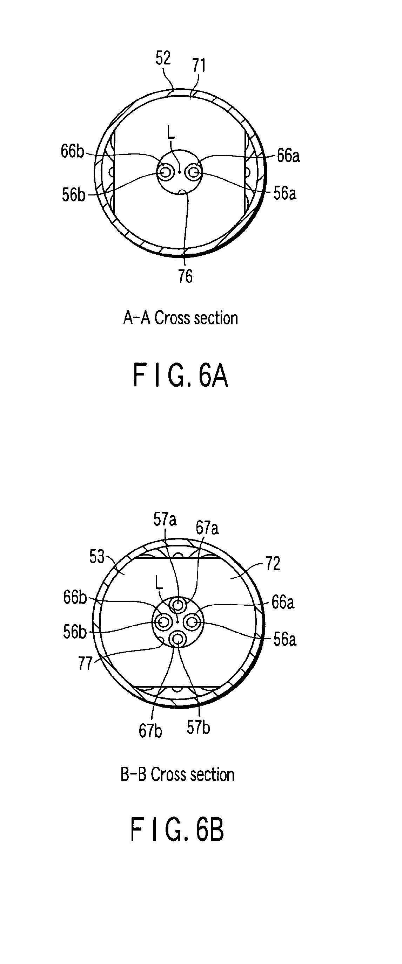

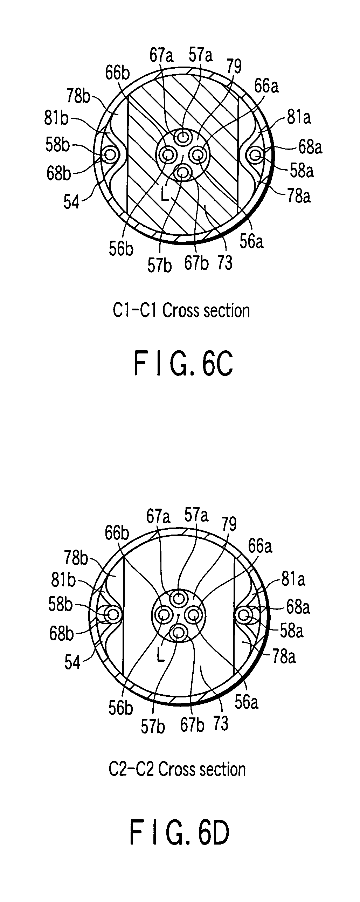

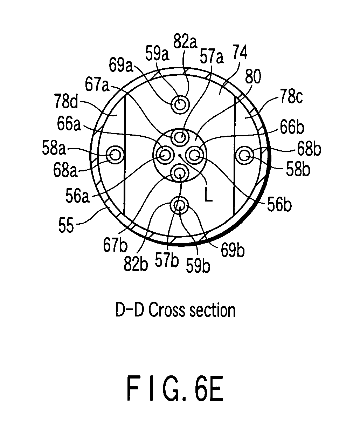

Next, how the manipulation wires and the guide sheaths are disposed in the bending pieces will be explained with reference to FIGS. 5A, 5B, 6A, 6B, 6C, 6D, and 6E.

The extreme ends of the guide sheaths 66a, 66b are fixed to a first wire guide 71 disposed in the second bending piece 52 and positioned to and supported by the second bending piece 52. The first wire guide 71 is formed of, for example, a sheet-shaped member as shown in FIGS. 5A, 5B and 6A. The first wire guide 71 is fixed to the inner wall of the second bending piece 52 by pins 60 at both the end edges thereof. An insertion hole 76, through which the contained members such as the guide sheaths 66a, 66b are inserted, is formed in a central portion of the first wire guide 71. The insertion hole 76 is formed in a circular shape around the center axis L. The extreme ends of the guide sheaths 66a, 66b are positioned and disposed in, for example, the right/left inner wall portions in the insertion hole 76, respectively and fixed to the portions by brazing or the like. Accordingly, the extreme ends of the guide sheaths 66a, 66b are disposed the same distance away from the center axis L. As described above, since the extreme ends of the guide sheaths 66a, 66b are disposed in the insertion hole 76, the extreme ends of the guide sheaths 66a, 66b are disposed in a region near the center in the bending portion 46. As a result, the manipulation wires 56a, 56b, which are guided by the guide sheaths 66a, 66b, are also positioned in a region near the center in the bending portion 46. That is, the first wire guide 71 plays a role as positioning/disposing means for positioning and disposing the guide sheaths 66a, 66b.

After the manipulation wires 56a, 56b project from the extreme ends of the guide sheaths 66a, 66b as shown in FIG. 5A, they enter the first bending piece 51 while extending, for example, right and left. The manipulation wire 56a is inserted into the cut and raised piece 70 in the right portion as described above. The manipulation wire 56b is inserted into the cut and raised piece 70 in the left portion as described above.

Note that although the extreme ends of the guide sheaths 66a, 66b are directly fixed to the first wire guide 71, they may be indirectly fixed to the first wire guide 71 using a connector such as a connecting ring, not shown.

As shown in FIGS. 5A, 5B and 6B, the extreme ends of the guide sheaths 67a, 67b are fixed to a second wire guide 72 disposed in the third bending piece 53 and positioned and supported thereby. The second wire guide 72 is formed of, for example, a sheet-shaped member. The second wire guide 72 is fixed to the inner wall of the third bending piece 53 at both the end edges thereof by pins 60. An insertion hole 77, through which the contained members such as the guide sheaths 66a, 66b, 67a, 67b are inserted, is formed in a central portion of the second wire guide 72. The insertion hole 77 is formed in a circular shape around the center axis L. The diameter of the insertion hole 77 is larger than that of the insertion hole 76. The extreme ends of the guide sheaths 67a, 67b are positioned and disposed in, for example, upper and lower inner wall portions in the insertion hole 77 and fixed to the portions by brazing or the like, respectively. Accordingly, the extreme ends of the guide sheaths 67a, 67b are disposed the same distance away from the center axis L. Further, the guide sheaths 67a, 67b are disposed further away from the center axis L than the guide sheaths 66a, 66b. That is, in the insertion hole 77, the guide sheaths 66a, 66b are disposed in a region nearer to a central portion in the bending portion 46 than the guide sheaths 67a, 67b. That is, the second wire guide 72 plays a role as positioning/disposing means for positioning and disposing the guide sheaths 67a, 67b.

The diameter of the insertion hole 77 is larger than that of the insertion hole 76, and the extreme ends of the guide sheaths 67a, 67b are disposed nearer to the inner wall of the insertion hole 77. Accordingly, as shown in FIG. 6B, the guide sheaths 67a, 67b are disposed further away from the center axis L than the guide sheaths 66a, 66b. In the second bending piece 52, the guide sheaths 66a, 66b are disposed and positioned by the first wire guide 71 so that they are disposed nearer to the center of the second bending piece 53 with respect to a direction vertical to the axial direction of the first rotation shaft 61. The guide sheaths 66a, 66b are guided to the third bending piece 53 in the above positional state. Therefore, the guide sheaths 66a, 66b are disposed inwards of the guide sheaths 67a, 67b in the third bending piece 53 with respect to a direction vertical to the axial direction of the second rotation shaft 62. That is, the guide sheaths 66a, 66b are disposed inwards of the guide sheaths 67a, 67b with respect to a direction vertical to the axial direction of the first and second rotation shafts 61, 62 (the direction of center axis L). Accordingly, the manipulation wires 56a, 56b are disposed inwards of the manipulation wires 57a, 57b in the direction of the center axis L.

After the manipulation wires 57a, 57b, which are guided by the guide sheaths 67a, 67b, project from the extreme ends of the guide sheaths 67a, 67b as shown in FIG. 5B, they enter the second bending piece 52 while extending, for example, up and down. The manipulation wire 57a is inserted into the cut and raised piece 70 in the upper portion as described above. The manipulation wire 57b is inserted into the cut and raised piece 70 in the lower portion as described above.

Note that although the extreme ends of the guide sheaths 67a, 67b are directly fixed to the second wire guide 72, they may be indirectly fixed to the second wire guide 72 using a connector such as a connecting ring, not shown.

The insertion hole 77 is formed in a circular shape, and the guide sheaths 66a, 66b are disposed nearer to the center axis L than the guide sheaths 67a, 67b. Accordingly, disposition of the guide sheaths 66a, 66b near the center axis L may result in formation of the right/left wall portions of the insertion hole 77 near the center axis L. In this case, the insertion hole 77 is formed not in a perfectly circular shape but in an oval shape which is longer in up and down directions. Accordingly, the right/left wall portions of the insertion hole 77 position and dispose the guide sheaths 66a, 66b inwards of the guide sheaths 67a, 67b. That is, the second wire guide 72 exhibits an effect as positioning/disposing means for positioning the guide sheaths 66a, 66b inwards of the guide sheaths 67a, 67b (positioning the manipulation wires 56a, 56b inwards of the manipulation wires 57a, 57b).

As described above, the extreme ends of the guide sheaths 67a, 67b are positioned and disposed in the upper/lower wall portions in the insertion hole 77. The guide sheaths 66a, 66b pass through the right/left regions of the insertion hole 77 in a state that they are not fixed to the second wire guide 72. Further, the guide sheaths 66a, 66b enter the insertion hole 77 after being regulated by the first wire guide 72. Accordingly, the guide sheaths 66a, 66b are disposed nearer to the inside of the central portion of the bending portion than the guide sheaths 67a, 67b.

As shown in FIGS. 5A, 5B, 6C and 6D, the third wire guide 73 is fixed to the fourth bending piece 54 and positioned and supported thereby. The third wire guide 73 is formed of a sheet-shaped member. The third wire guide 73 is fixed to the inner wall of the fourth bending piece 54 at both the end edges thereof by pins 60. As shown in FIGS. 6C and 6D, the right/left portions of the third wire guide 73 are eliminated so that the third wire guide 73 is away from the inner wall of the fourth bending piece 54. Spaces 78a, 78b are formed in the eliminated portions to position and dispose the extreme ends of the guide sheaths 68a, 68b. That is, the spaces 78a, 78b play a role as positioning/disposing means for positioning and disposing the extreme ends of the guide sheaths 68a, 68b. Note that the extreme ends of the guide sheaths 68a, 68b are connected to, for example, the inner wall of the fourth bending piece 54 in the spaces 78a, 78b.

Specifically, as shown in FIG. 6D, a cut and raised piece 81a, which projects inside of the fourth bending piece 54, is formed in the right inner wall of the fourth bending piece 54. Further, a cut and raised piece 81b, which projects inside of the fourth bending piece 54, is formed in the left inner wall of the fourth bending piece 54. The extreme end of the guide sheath 68a is inserted into the cut and raised piece 81a and fixed thereto by brazing. The extreme end of the guide sheath 68b is inserted into the cut and raised piece 81b and fixed thereto by brazing.

Further, as shown in FIG. 6C, the portion other than the extreme end (for example, the intermediate end) of the guide sheath 68a is fixed to the right inner wall of the third wire guide 73, and the portion other than the extreme end (for example, the intermediate end) of the guide sheath 68b is fixed to the left inner wall of the third wire guide 73.

Note that the extreme ends of the guide sheaths 68a, 68b may be fixed to the third wire guide 73 as shown in FIG. 5A.

Further, although the extreme ends of the guide sheaths 68a, 68b are directly fixed to the fourth bending piece 54 or to the third wire guide 73, they may be indirectly connected to the fourth bending piece 54 or to the third wire guide 73 using a connector such as a connection ring, not shown.

Further, as shown in FIGS. 6C and 6D, an insertion hole 79, into which the contained members such as the guide sheaths 66a, 66b, 67a, 67b are inserted, is formed in the central portion of the third wire guide 73. The insertion hole 79 is formed in a circular shape around the center axis L. The diameter of the insertion hole 79 is larger than the diameter of the insertion hole 76 and is the same as or larger than the diameter of the insertion hole 77. The guide sheaths 66a, 66b, which are previously positioned by the first wire guide 71, and the guide sheaths 67a, 67b, which are previously positioned by the second wire guide 72, are inserted into the insertion hole 79, respectively in the states in which they are positioned. That is, the third wire guide 73 gathers the guide sheaths 66a, 66b and the guide sheaths 67a, 67b near the central region of the bending portion 46.

Further, the guide sheaths 66a, 66b and the guide sheaths 67a, 67b are disposed inwards of the guide sheaths 68a, 68b with respect to a direction vertical to the axial direction of the first and second rotation shafts 61, 62 (the direction of the center axis L). That is, in the direction of the center axis L, the manipulation wires 56a, 56b and the manipulation wires 57a, 57b are disposed inwards of the manipulation wires 58a, 58b. As described above, the third wire guide 73 plays a role as positioning/disposing means for positioning the guide sheaths 66a, 66b and the guide sheaths 67a, 67b (the manipulation wires 56a, 56b and the manipulation wires 57a, 57b) inwards of the guide sheaths 68a, 68b (the manipulation wires 58a, 58b).

On the same plane in a direction vertical to the axial direction of the third rotation shaft 63, the guide sheaths 66a, 66b are interposed between the guide sheath 68a and the guide sheath 68b as shown in FIGS. 6C and 6D. Further, the guide sheaths 66a, 66b and the guide sheaths 67a, 67b are disposed nearer to the central region in the vicinity of the center axis L than the guide sheaths 68a, 68b.

The insertion hole 79 is formed in a circular shape, and the guide sheaths 66a, 66b are disposed nearer to the center axis L than the guide sheaths 67a, 67b. Accordingly, disposition of the guide sheaths 66a, 66b near the center axis L may result in formation of the right/left wall portions of the insertion hole 79 near the center axis L. In this case, the insertion hole 79 is formed not in a perfectly circular shape but in an oval shape which is longer in up and down directions. Accordingly, the right/left wall portions of the insertion hole 79 position and dispose the guide sheaths 66a, 66b inwards of the guide sheaths 67a, 67b. That is, the third wire guide 73 exhibits an effect of positioning the guide sheaths 66a, 66b.

As shown in FIGS. 5A, 5B and 6E, a fourth wire guide 74 is fixed to the fifth bending piece 55. The fourth wire guide 74 is formed of a sheet-shaped member like the wire guide 71 and the like. The fourth wire guide 74 is fixed to the inner wall of the fifth bending piece 55 at both the end edges thereof by pins 60. As shown in FIG. 6E, the right/left portions of the fourth wire guide 74 are eliminated so that the fourth wire guide 74 is away from the inner wall of the fifth bending piece 55. Spaces 78c, 78d, into which the guide sheaths 68a, 68b are inserted, are formed in the eliminated portions. The guide sheaths 68a, 68b are not fixed to the fourth wire guide 74 and pass through the spaces 78c, 78d in a state that they are positionally regulated by the third wire guide 73. The guide sheaths 68a, 68b are positionally regulated by the fourth wire guide 74 so that they are prevented from entering the center of the fifth bending piece 55.

Further, as shown in FIG. 6E, an insertion hole 80, into which the contained members such as the guide sheaths 66a, 66b and the guide sheaths 67a, 67b are inserted, is formed in the center of the fourth wire guide 74. The insertion hole 80 is formed in a circular shape around the center axis L. The diameter of the insertion hole 80 is larger than the diameter of the insertion hole 76 and is the same as or larger than the diameters of the insertion holes 77, 79. The guide sheaths 66a, 66b and the guide sheaths 67a, 67b are movably inserted into the insertion hole 80. With this configuration, the fourth wire guide 74 plays a role of gathering the guide sheaths 66a, 66b, 67a, 67b to the vicinity of the central region of the bending portion 46. That is, the fourth wire guide 74 also plays a role as positioning/disposing means of the guide sheaths 66a, 66b and the guide sheaths 67a, 67b.

On the same plane in the axial direction of the fourth rotation shaft 64, the guide sheaths 66a, 66b are interposed between the guide sheath 68a and the guide sheath 68b as shown in FIG. 6E. Further, on the same plane in a direction vertical to the axial direction of the fourth rotation shaft 64, the guide sheaths 67a, 67b are interposed between the guide sheath 69a and the guide sheath 69b. The guide sheaths 66a, 66b and the guide sheaths 67a, 67b are disposed nearer to the central region in the vicinity of the center axis L than the guide sheaths 68a, 68b and the guide sheaths 69a, 69b.

The insertion hole 80 is formed in a circular shape, and the guide sheaths 66a, 66b are disposed nearer to the center axis L than the guide sheaths 67a, 67b. Accordingly, disposition of the guide sheaths 66a, 66b near the center axis L may result in formation of the right/left wall portions of the insertion hole 80 near the center axis L. In this case, the insertion hole 80 is formed not in a perfectly circular shape but in an oval shape which is longer in up and down directions. Accordingly, the right/left wall portions of the insertion hole 80 position and dispose the guide sheaths 66a, 66b inwards of the guide sheaths 67a, 67b. That is, the fourth wire guide 74 exhibits an effect of positioning the guide sheaths 66a, 66b.

A pair of fixing holes 82 (82a, 82b) are formed in both the upper/lower ends of the fourth wire guide 74. The fixing holes 82a, 82b are disposed vertically symmetrically with respect to the insertion hole 80. The fixing hole 82a is disposed above the insertion hole 80, and the fixing hole 82b is disposed below the insertion hole 80. In this case, the fixing holes 82a, 82b are disposed outwards of the guide sheaths 68a, 68b. That is, the fixing holes 82a, 82b are disposed further away from the center axis L than the guide sheaths 68a, 68b. The extreme end of the guide sheath 69a is inserted into the fixing hole 82a and fixed thereto by, for example, brazing. The extreme end of the guide sheath 69b is inserted into the fixing hole 82b and fixed thereto by, for example, brazing. Accordingly, as shown in FIG. 6E, the extreme ends of the guide sheaths 69a, 69b are disposed near the inner wall of the fifth bending piece 55 as well as outwards of the guide sheaths 68a, 68b.

That is, the guide sheaths 68a, 68b are disposed inwards of the guide sheaths 69a, 69b with respect to a direction vertical to the axial direction of the third and fourth rotation shafts 63, 64 (in the direction of the center axis L). In other words, the manipulation wires 58a, 58b are disposed inwards of the manipulation wires 59a, 59b. Further, the guide sheaths 66a, 66b and the guide sheaths 67a, 67b are disposed inwards of the guide sheaths 68a, 68b and the guide sheaths 69a, 69b. In other words, the manipulation wires 56a, 56b and the manipulation wires 57a, 57b are disposed inwards of the manipulation wires 58a, 58b and the manipulation wires 59a, 59b.

That is, the fourth wire guide 74 plays a role as positioning/disposing means for positioning the guide sheaths 66a, 66b, the guide sheaths 67a, 67b, and the guide sheaths 68a, 68b (the manipulation wires 56a, 56b, the manipulation wires 57a, 57b, and the manipulation wires 58a, 58b) inwards of the guide sheaths 69a, 69b (the manipulation wires 59a, 59b).

As described above, the wire guides play the role of the positioning/disposing means for specifying the positions of the guide sheaths and at the same time play the role as the positioning/disposing means for determining the positions of the manipulation wires which are individually guided by the guide sheaths. Since the positions of the guide sheaths are determined in the bending portion 46, the contained members including the guide sheaths are prevented from interfering with each other. Further, the manipulation wires inserted into the guide sheaths are prevented from being in direct contact with the other manipulation wires and the contained members thanks to the guide sheaths. Accordingly, interference between the manipulation wires and between the manipulation wires and the contained members is reduced.

Further, as shown in FIGS. 6C and 6D, a first guide sheath group of the guide sheaths 66a, 66b and the guide sheaths 67a, 67b is positioned inwards of a second guide sheath group of the guide sheaths 68a, 68b and gathered to the central region of the bending portion 46 positioned inwards of the second guide sheath group. The guide sheaths 66a, 66b and the guide sheaths 68a, 68b are positioned on the same radius line passing through the center axis L and overlap inside and outside on the radius line.

Further, as shown in FIG. 6E, the first guide sheath group of the guide sheaths 66a, 66b and the guide sheaths 67a, 67b are positioned inwards of a second guide sheath group of the guide sheaths 68a, 68b and the guide sheaths 69a, 69b and gathered to the central region of the bending portion 46 positioned inwards of the second guide sheath group. The guide sheaths 66a, 66b and the guide sheaths 68a, 68b are positioned on the same radius line passing through the center axis L and positioned inside and outside on the radius line. The guide sheaths 67a, 67b and the guide sheaths 69a, 69b are positioned on the same radius line passing through the center axis L and overlap inside and outside on the radius line.

As shown in FIG. 4, the fifth bending piece 55 is a bending piece positioned at the most extreme base end of the bending portion 46. That is, it is possible to assume that the fifth bending piece 55 is the base end portion of the bending portion 46. A connector member 94 such as a connection ring is disposed in the extreme end of the flexible tube 45. The fifth bending piece 55 is coupled with the connector member 94. Further, the fifth bending piece 55 may be rotatably coupled with the connector member 94. In this mode, it is also possible to assume that the connector member 94 is the base end portion of the bending portion 46.

Next, the angular relationship under which the respective bending pieces mutually rotate will be explained with reference to FIGS. 7A and 7B.

The end faces 91, which confront each other (which are adjacent to each other) in adjacent bending pieces, form a gap 90. The gap 90 expands in a fan-shape at an angle θ around the axis of a rotation shaft. In more detail, lines extending from the end faces 91 intersect on the axis of the rotation shaft. Accordingly, the respective end faces 91 are formed as linear end edges passing through the rotation axis, respectively. Then, the gap 90 is formed by the two end faces 91 confronting each other and expanding in a fan-shape at an angle θ about an intersecting point (axis of the rotation shaft).

Note that the extended lines need not necessarily intersect on the axis of the rotation shaft, and the respective end faces 91 may not be formed as linear end edges passing through the rotation axis, respectively. In this case, the gap 90, which expands in a fan-shape at the angle θ, may be preferably formed by the lines which pass through ends (apexes) 91a, which are positioned at the most external sides of the end faces 91, and the axis of the rotation shaft.

Note that the sum of the angles θ of the gaps 90 of at least two adjacent bending pieces in the bending pieces rotating in the same direction is set to 90° or more. As shown in, for example, FIGS. 7A and 7B, the sum of the rotatable angle θ1 of the gap 90 between the bending pieces 51, 52 rotating in the same direction and the rotatable angle θ2 of the gap 90 between the bending pieces 53, 54 rotating in the same direction is set to 90° or more.

As described above, the rotatable angle θ of the multijointed bending piece may be preferably allocated not only to one gap 90 but also to the gaps 90 between the bending pieces rotating in the same direction (a plurality of adjacent gaps 90). With this configuration, it is not necessary to increase the angle θ in one gap 90. Accordingly, the maximum angle θ formed by one gap 90 is reduced. As a result, the amount of rotation of a bending piece in one gap 90 is reduced. Thus, when a bending operation causes the contained members such as the manipulation wires and the guide sheaths to traverse the gap 90, they are less likely to be caught by the gap 90.

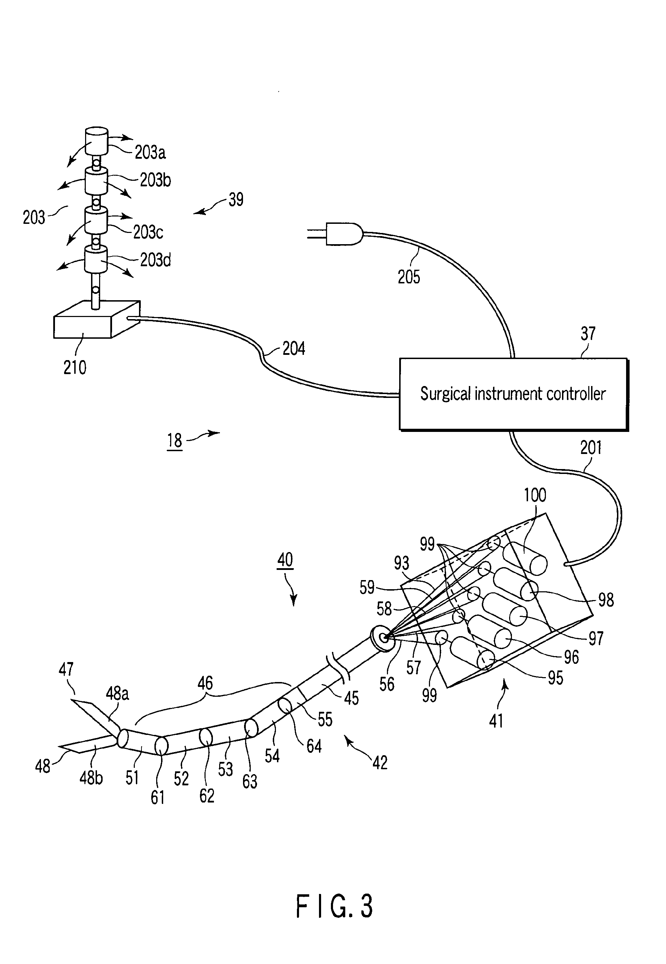

As shown in FIG. 3, the manipulation unit 41 is provided with a bending portion manipulation mechanism and a surgical portion manipulation mechanism. The bending portion manipulation mechanism is provided with drive motors 95, 96, 97, 98 for pushing and pulling the manipulation wires 56, 57, 58, 59, respectively. Further, the surgical portion manipulation mechanism is provided with a drive motor 100 for pushing and pulling the manipulation wire 93. The manipulation wires 56, 57, 58, 59 correspond to the bending pieces (targets to be rotated) 51, 52, 53, 54 and execute rotating manipulations. The manipulation wire 93 manipulates the grip forceps 48.

Pulleys 99 are attached to drive shafts of the drive motors 95, 96, 97, 98, 100, respectively. The respective drive shafts may be coupled with the respective pulleys 99 through reducers (not shown). The manipulation wires 56, 57, 58, 59, 93 are trained round the respective pulleys 99. The drive motors 95, 96, 97, 98, 100 are individually driven, respectively, and when the pulleys 99 are rotated, the manipulation wires 56, 57, 58, 59, 93 trained around the pulleys 99 are pushed and pulled.

Although the bending portion manipulation mechanism and the surgical portion manipulation mechanism use transmission mechanisms making use of the pulleys 99, they may use, for example, a gear mechanism and the like making use of a pinion gear and a rack. Further, the bending portion manipulation mechanism and the surgical portion manipulation mechanism may use other types of drive actuators in place of the drive motors 95, 96, 97, 98, 100.

As shown in FIGS. 2 and 3, the manipulation unit 41 is connected to the surgical instrument controller 37 through a cable 201. The bending manipulation unit 39 as the manipulation input unit is connected to the surgical instrument controller 37 through a cable 204. In FIG. 3, the surgical instrument controller 37 is provided with a power supply cord 205.

The bending manipulation unit 39 includes a joystick (manipulation input unit) 203 for instructing a position and an attitude of the surgical instrument 40. The joystick 203 includes four joystick switches 203a, 203b, 203c, 203d continuously connected in four stages. The joystick switches 203a, 203b, 203c, 203d are attached to a manipulation box 210.

When the joystick switches 203a, 203b, 203c, 203d are selectively manipulated, the drive motors 95, 96, 97, 98 are individually driven corresponding to the manipulation. With this manipulation, the bending pieces 51, 52, 53, 54 are individually and independently driven in up, down, right, and left directions to thereby bend respective joint portions.

The surgical instrument extreme end movement controller 18 can move the extreme end portion 47 to a desired position by the movement according to the manipulation of the joystick 203. That is, the surgical instrument extreme end movement controller 18 constitutes the surgical instrument 40 which is arranged as a master/slave type and driven electrically. Note that, when the joystick 203 is manipulated by an operator and the like after a control for moving the surgical instrument 40 is set, preference is given to an instruction for manipulating the joystick 203.

As shown in FIG. 2, the surgical instrument controller 37 is provided with a function control input portion 121 for inputting an instruction output from the joystick 203, a condition for controlling the function of the joystick 203, and the like, a motor driver (surgical instrument drive controller) 122 for controlling the drive of the drive motors 95, 96, 97, 98, and a motor unit communication unit 123 connected to the surgical instrument drive unit 38 through the cable 201 for executing communication with the surgical instrument drive unit 38.

The surgical instrument controller 37 transmits a control signal for driving the drive motors 95, 96, 97, 98 in response to the manipulation of the joystick 203 executed by the operator to the motor driver 122 and rotates the drive motors 95, 96, 97, 98. Encoders (not shown) are mounted on the drive motors 95, 96, 97, 98 to measure the number of revolutions thereof. The encoders feedback-control the drive motors 95, 96, 97, 98 by generating signals according to the number of revolutions and transmitting the signals to the motor driver 122.

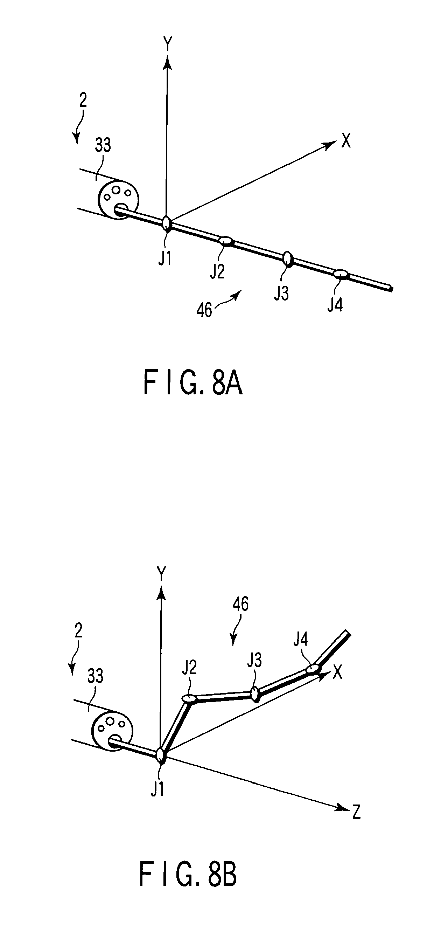

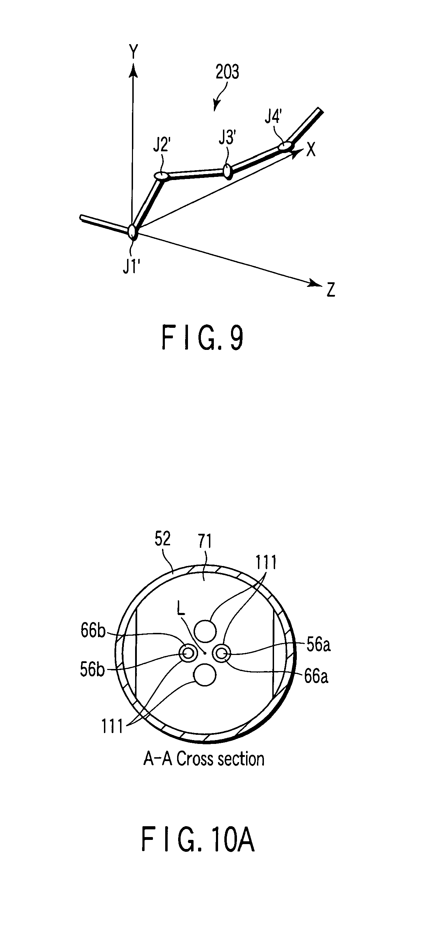

The relation between a multijointed structure in the bending portion 46 and the joystick 203 will be explained with reference to FIGS. 8A, 8B and 9.

As shown in FIG. 8A, in a state that all the joint portions in the bending portion 46 project from the extreme end portion 33, the joints disposed from the manipulation unit side (base end side) to the extreme end side are sequentially referred to as J1, J2, J3, J4. A coordinate system is set using the joint J1 disposed nearest to the manipulation unit side as a reference. In the coordinate system, a Y-axis direction agrees with a vertical direction of the image pickup device. It is assumed that the joints J1, J3 are bent about an X-axis, and the joints J2, J4 are bent about a Y-axis.

As shown in FIG. 9, the joystick 203 (manipulation input unit) includes joints J1', J2', J3', J4' which have the same structures as those of the joint J1 and the joints J2, J3, J4 located nearer to the extreme end side than the joint J1.

The number of the joints and the bending directions of the joystick 203 are the same as those of the bending portion 46. The lengths of respective rods of the joystick 203 are set to values multiplied by an appropriate coefficient k so that the operator can easily manipulate them. When, for example, k = 10 and the length of each rod of the surgical instrument 40 is 3 mm, the length of each rod of the joystick 203 (manipulation input unit) is set to 30 mm. Encoders (not shown) are assembled to the joints J1', J2', J3', J4' to measure bent angles. The information of the bent angles measured by the encoders is sent to the surgical instrument controller 37. The surgical instrument controller 37 generates drive signals corresponding to the angle information (the joints J1', J2', J3', J4') and bends the joints J1, J2, J3, J4 by rotating the drive motors 95, 96, 97, 98, respectively. When the joints J1', J2', J3', J4' are bent as shown in, for example, FIG. 9, the joints J1, J2, J3, J4 are bent as shown in FIG. 8B.

Since the bending portion 46 has a plurality of joints, the extreme end of the surgical instrument 40 can be moved to an arbitrary position and an arbitrary attitude so that an affected area can be more easily cut out and exfoliated than ever before. Further, since the joint structure of the surgical instrument 40 is caused to equally correspond to that of the manipulation input unit, the operator can easily operate the surgical instrument having a plurality of joints.

Further, the drive motor 100 also has a motor driver, a motor unit communication unit, and the like similarly to the drive motors 95, 96, 97, 98. The grip forceps 48 is manipulated by manipulating a manipulation body such as a handle (function control/input unit) 125 disposed in the manipulation unit 41 and the like.

Note that the manipulation input unit may be preferably provided with a first manipulation switch corresponding to the first bending piece 51, a second manipulation switch corresponding to the second bending piece 52, a third manipulation switch corresponding to the third bending piece 53, and a fourth manipulation switch corresponding to the fourth bending piece 54. When, for example, the first manipulation switch is depressed, the first bending piece 51 is bent. Further, the manipulation unit 41 may be preferably provided with a switch device (manipulation switch) for the bending manipulation. The manipulation input unit may use a pen type input unit for inputting a three-dimensional position.

Next, an operation when the surgical instrument 40 is used will be explained.

First, as shown in FIG. 2, the insertion portion 21 is inserted into a body cavity, and the insertion portion 42 is inserted from the insertion port 28 into the insertion channel in this state. The extreme end portion 47 and the bending portion 46 project from the channel port 36 into the body cavity. Then, a work for gripping an affected area and the like in the body cavity is executed using the grip forceps 48 while observing them by the endoscope 2.

In this case, the bending portion 46 can be bend to an appropriate multijointed bent shape according to the state in the body cavity and the surgical procedure. That is, when the joystick 203 is manipulated and the bending pieces 51, 52, 53, 54 are individually rotated, the bending portion 46 is bend into an appropriate shape.

When, for example, the drive motor 95 is driven, the manipulation wires 56a, 56b trained around the pulley 99 in the drive motor 95 are pushed and pulled. With this operation, the first bending piece 51 is independently rotated. When the drive motor 96 is driven, the manipulation wires 57a, 57b trained around the pulley 99 in the drive motor 96 are pushed and pulled. With this operation, the second bending piece 52 is independently rotated. When the drive motor 97 is driven, the manipulation wires 58a, 58b trained around the pulley 99 in the drive motor 97 are pushed and pulled. With this operation, the third bending piece 53 is independently rotated. Further, when the drive motor 98 is driven, the manipulation wires 59a, 59b trained around the pulley 99 in the drive motor 98 are pushed and pulled. With this operation, the fourth bending piece 54 is independently rotated.

Accordingly, when the joystick 203 is appropriately operated, the bending pieces 51, 52, 53, 54 are independently rotated, and the bending portion 46 is bent. The bending portion 46 can even be bend in a complex shape by adjusting the direction in which the joystick 203 is rotated and the amount of rotation thereof.

As described above, in the embodiment, since the manipulation wires are disposed in the plurality of bending pieces, an arbitrary bending piece can be independently rotated. Accordingly, in the embodiment, since the bending mechanism has a plurality of degrees of freedom, a work can be executed even in a narrow region such as a body cavity.

In more detail, in the embodiment, since the bending pieces 51, 52, 53, 54 can be independently rotated (bend), the bending portion 46 can be partially bend also in a different direction. Thus, in the embodiment, the bending portion 46 can be bend into an appropriate shape according to a state of use. As a result, since the degree of freedom of bending of the bending portion 46 is increased, it is possible in the embodiment to easily execute even a complex work in a narrow body cavity region as compared with a case in which the bending portion 46 is uniformly bend. Further, in the embodiment, since the attitude of the bending portion 46 can be easily bend so that it does not disturb another surgical instrument or observation with the endoscope 2, the workability of the surgical instrument 40 can be increased.

Further, in the embodiment, since the bending pieces 51, 52, 53, 54 can be independently bend, respectively, the joints of the bending pieces 51, 52, 53, 54 can be stably bend without being largely affected by the other bend portions. Accordingly, the embodiment can easily and securely execute a desired surgical with the surgical instrument 40.

Further, in the embodiment, the manipulation wire connected to a bending piece disposed on the extreme end side is disposed inwards of the manipulation wire connected to a bending piece disposed on the base end side. With this configuration, the manipulation force of the manipulation wire connected to the bending piece disposed on the extreme end side and having a large frictional resistance due to the bending of the bending piece can be reduced. Accordingly, in the embodiment, since the manipulation force required by the manipulation wires can be reduced, made uniform, and stabilized, a manipulation can be smoothly executed by the manipulation wires.

In more detail, the manipulation wires 56a, 56b guided by the guide sheaths 66a, 66b are disposed nearer to the center of the bending portion 46 than the manipulation wires 57a, 57b guided by the guide sheaths 67a, 67b. Accordingly, when the bending pieces 51, 52, 53, 54 are bend, the frictional force which the manipulation wires 56a, 56b receive from the contained members becomes smaller than the frictional force which the manipulation wires 57a, 57b receive from the contained members. Accordingly, in the embodiment, the manipulation force required by the manipulation wires 56a, 56b can be reduced, made uniform, and stabilized, and the manipulation can be smoothly executed by the manipulation wires 56a, 56b.

Further, the first and second wire guides 71, 72 dispose the manipulation wires 56a, 56b, the guide sheaths 66a, 66b, the manipulation wires 57a, 57b, and the guide sheaths 67a, 67b near the region of the central portion of the bending pieces 52, 53, 54, 55 (bending portion 46). The third and fourth wire guides 73, 74 dispose the manipulation wires 58a, 58b, the guide sheaths 68a, 68b, the manipulation wires 59a, 59b, and the guide sheaths 69a, 69b to the peripheral region in the bending pieces 52, 53, 54, 55 (bending portion 46).

That is, in the embodiment, the manipulation wire for bending one bending piece disposed in the extreme end side and the guide sheath for guiding the manipulation wire are disposed more inwards of the bending portion 46 than the manipulation wire for bending the other bending piece disposed nearer to the base end side than the one bending piece and the guide sheath for guiding the manipulation wire.

Accordingly, the embodiment can obtain the following operation/working effect. When the plurality of bending pieces are bent, a manipulation wire for bending a bending piece disposed nearer to the extreme end side receives a larger frictional resistance from the contained members. However, the manipulation wire for bending the bending piece disposed in the extreme end side is disposed further inwards of the bending portion 46 than the manipulation wire for bending the bending piece disposed in the base end side as described above. Thus, the frictional resistance which the manipulation wire for bending the bending piece on the extreme end side receives is reduced. Thus, according to the embodiment, the manipulation force required by the manipulation wires can be reduced, made uniform, and stabilized, and the manipulation can be smoothly executed by the manipulation wires. Further, in the embodiment, since many manipulation wires can be disposed compactly without being entangled with each other, the diameter of a bending mechanism portion can be easily reduced.

In general, when a plurality of bending pieces are bend, respectively, the path of a manipulation wire for manipulating a bending piece located nearer to an extreme end side changes to a greater extent. Since the frictional resistance is increased by such change, the loss of manipulation force is increased. As a result, the manipulation force for manipulating the manipulation wire for bending the bending piece disposed on the extreme end side is increased. However, in the embodiment, the manipulation wire for bending the bending piece disposed on the extreme end side is disposed in the region (in the vicinity of the central region in the bending portion 46) inwards of the manipulation wire for bending the bending piece disposed on the base end side. Accordingly, the embodiment can reduce the loss of the manipulation force by suppressing an increase of the frictional resistance of the manipulation wire on the extreme end side. Further, in the embodiment, since the path through which the manipulation wire passes is less changed even if the bending portion 46 is bend, a rotating manipulation can be executed lightly. Further, in the embodiment, when the bending portion 46 is bend, since a wasteful motion is suppressed, the change of the manipulation force can be also suppressed. Accordingly, the embodiment can securely transmit the manipulation force up to the bending piece on the extreme end side. Further, the embodiment can independently manipulate many bending pieces.

Further, in general, many manipulation wires and many guide sheaths are disposed in the narrow bending portion 46. The embodiment can dispose the many manipulation wires and the many guide sheaths in the narrow bending portion 46 compactly by the positioning/disposing means. Accordingly, in the embodiment, the manipulation wires are less entangled in the bending portion 46 and the occurrence of interference of the manipulation wires with each other can be reduced.

Further, in the embodiment, a guide sheath, which guides a manipulation wire for rotating a bending piece, is connected to a bending piece located just behind the above bending piece (on the base end side). Thus, the embodiment can maximize the efficacy of the wire guide function achieved by the guide sheath. Further, the region in which the manipulation wires are separately exposed can be reduced. Accordingly, the embodiment can avoid any reduction in the wire guide functionality. Further, when, for example, the insertion portion 42 itself is twisted, the embodiment can alleviate the effect of the twist on the wire guide function of the guide sheaths.

Further, in the embodiment, the guide sheaths may be formed of an intimately wound metal coil. With this configuration, the embodiment can sufficiently withstand any abrupt rotating and bending actions of the bending pieces.

Note that, although the embodiment uses the guide sheaths and disposes the guide sheaths at predetermined positions by use of the wire guides, it can also dispose the manipulation wires at the predetermined positions by use of the wire guides without using the guide sheaths. In, for example, FIGS. 5A and 5B, the embodiment can also directly position the manipulation wires in a state that the guide sheaths are omitted.

Further, in the embodiment, the rotation shaft is not limited to the rivet-shaped portion and may be arranged as, for example, a pin-shaped portion, a locking portion for rotatably locking the bending pieces with each other, and the like.

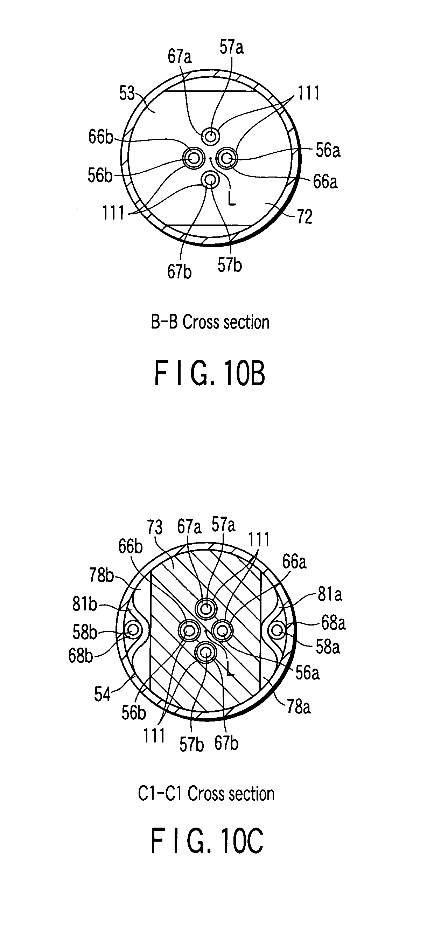

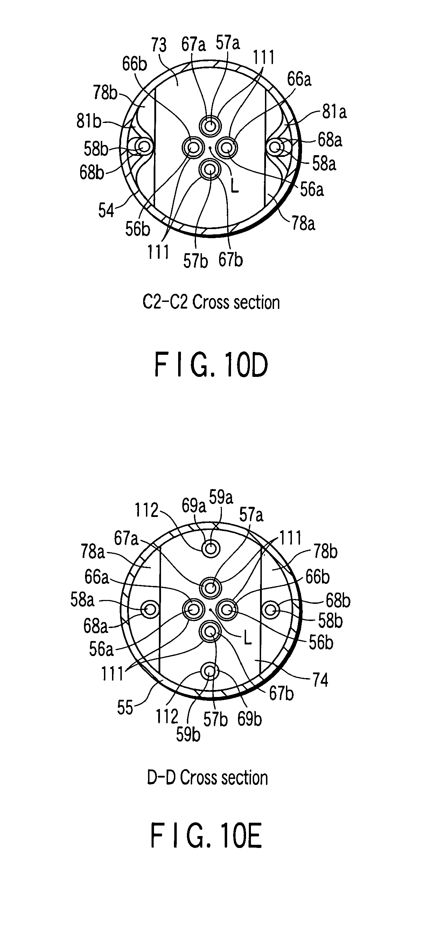

Next, a modification of the wire guides in the embodiment described above will be explained with reference to FIGS. 10A, 10B, 10C, 10D and 10E.

Wire guides 71, 72, 73, 74 are composed of a sheet-shaped member, and a plurality of lumens 111 are formed to the wire guides 71, 72, 73, 74. The wire guides 71, 72, 73, 74 position and dispose guide sheaths 66a, 66b, 67a, 67b, 69a, 69b by the lumens 111 and 112.

As shown in FIG. 10A, a first wire guide 71 is disposed in a second bending piece 52. The four lumens 111 are formed on the top, bottom, right, and left of the center of the first wire guide 71. The right and left lumens 111 are disposed at the same distance from the center axis L. The upper and lower lumens 111 are disposed further away from the center axis L than the right and left lumens 111. Accordingly, the right and left lumens 111 are disposed nearer to the center axis L than the upper and lower lumens 111.

The extreme ends of the guide sheaths 66a, 66b are inserted into the right and left lumens 111 and fixed thereto by brazing. The upper and lower lumens 111 are used as paths for guiding other contained members and the like from, for example, the upper and lower lumens 111 to an extreme end side. The upper and lower lumens 111 are used as paths for guiding the other contained members and the like to the central region.

As shown in FIG. 10B, the second wire guide 72 is disposed in a third bending piece 53. The four lumens 111 are formed on the top, bottom, right, and left of the center of the second wire guide 72. The right and left lumens 111 are disposed in the same distance from the center axis L. The upper and lower lumens 111 are disposed further away from the center axis L than the right and left lumens 111. Accordingly, the right and left lumens 111 are disposed nearer to the center axis L than the upper and lower lumens 111.

The guide sheaths 66a, 66b are inserted into the right and left lumens 111 so as to move forward and rearward therein. The extreme ends of the guide sheaths 67a, 67b are inserted into the upper and lower lumens 111 and fixed thereto by brazing.

Accordingly, the distance from the guide sheaths 66a, 66b to the center axis L in the axial direction of the second rotation shaft 62 is smaller than that from the guide sheaths 67a, 67b to the center axis L in a direction vertical to the axial direction of the second rotation shaft 62. Accordingly, the guide sheaths 66a, 66b are disposed inwards of the guide sheaths 67a, 67b.

As shown in FIGS. 10C and 10D, the third wire guide 73 is disposed in a fourth bending piece 54. The four lumens 111 are formed on the top, bottom, right, and left of the center of the third wire guide 73. The four lumens 111 are disposed in the same manner as the lumens 111 disposed in the first wire guide 71 and the second wire guide 72.

The guide sheaths 66a, 66b are inserted into the right and left lumens 111 so as to move forward and rearward therein. The guide sheaths 67a, 67b are inserted into the upper and lower lumens 111 so as to move forward and rearward therein. The extreme ends of the guide sheaths 68a, 68b are fixed to cut and raised pieces 81a, 81b, or the third wire guide 73 in spaces 78c, 78d as in the above embodiment.

As shown in FIG. 10E, the fourth wire guide 74 is disposed in a fifth bending piece 55. The four lumens 111 are formed on the top, bottom, right, and left of the center of the fourth wire guide 74. The four lumens 111 are disposed in the same manner as the lumens 111 disposed in the third wire guide 73 described above.

The guide sheaths 66a, 66b are inserted into the right and left lumens 111 so as to move forward and rearward therein. The guide sheaths 67a, 67b are inserted into the upper and lower lumens 111 so as to move forward and rearward therein. The guide sheaths 68a, 68b are inserted into the spaces 78c, 78d as in the above embodiment.

The lumens 112 for attaching the extreme ends of the guide sheaths 69a, 69b are formed in the upper and lower end portions of the fourth wire guide 74. The distance from the lumens 112 to the center axis L is longer than the distance in a radius direction from the guide sheaths 68a, 68b to the center axis L. That is, the lumens 112 are disposed outwards of the guide sheaths 68a, 68b. Since the remaining configuration of the modification is approximately the same as that of the embodiment described above, the explanation thereof is omitted.

In the modification, the positions of the guide sheaths 66a, 66b, 67a, 67b, 69a, 69b are regulated by the lumens 111, 112. Accordingly, in the modification, since the manipulation wires 56a, 56b, 57a, 57b and the wire guides 71, 72, which are disposed in the vicinity of the center, can be simply attached to predetermined positions, they can be securely and accurately disposed.

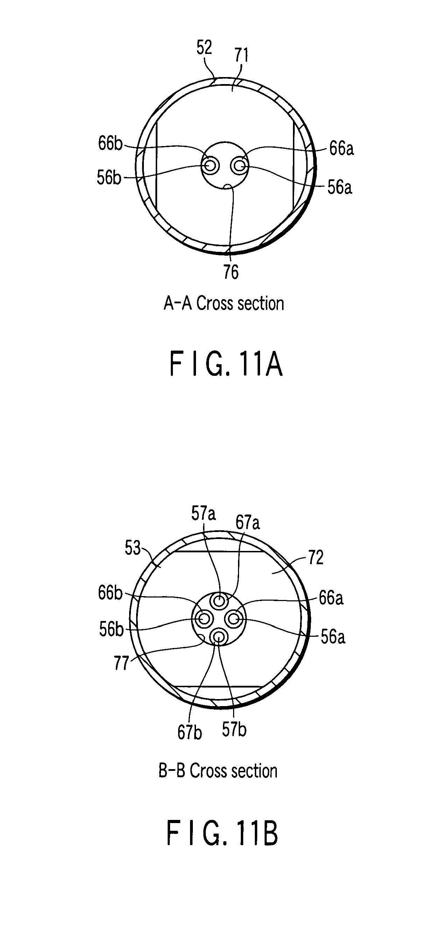

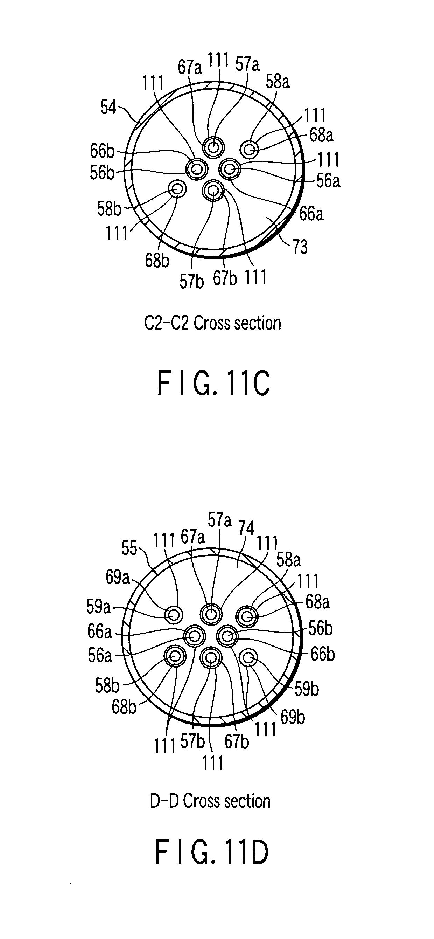

Next, another modification of the positioning/disposing means in the embodiment described above will be explained with reference to FIGS. 11A, 11B, 11C and 11D.

In the modification, the guide sheaths 66a, 66b, 67a, 67b are disposed in the central region of the bending portion 46 by the first wire guide 71 and the second wire guide 72. This is the same as the positioning/disposing means in the first embodiment.

The third and fourth wire guides 73 and 74 are arranged as described below.

Guide sheaths having flexibility are disposed in the third and fourth wire guides 73 and 74. Alternatively, lumens 111 for inserting the guide sheaths are formed to the third and fourth wire guides 73 and 74. The lumens are obliquely arranged to a sheet member for forming the wire guides. This point is different in respect of the embodiment described above.

That is, as shown in FIG. 11C or 11D, the guide sheaths 68a, 68b, 69a, 69b having flexibility are obliquely arranged by being offset by a predetermined angle, respectively in a peripheral region disposed outwards of the guide sheaths 66a, 66b, 67a, 67b disposed in an inner central region.

In this case, the guide sheaths are not disposed up, down, right and left around the center axis L unlike the embodiment described above. Accordingly, in the modification, since the respective guide sheaths are obliquely disposed and the contained members are disposed compactly by making use of the narrow space in the bending portion 46, the diameter of the bending portion 46 can be reduced.

Note that, although the guide sheaths are positioned without using wire guides as shown in FIGS. 11C and 11D, they may be disposed by the positioning/disposing means as described in the embodiment described above.

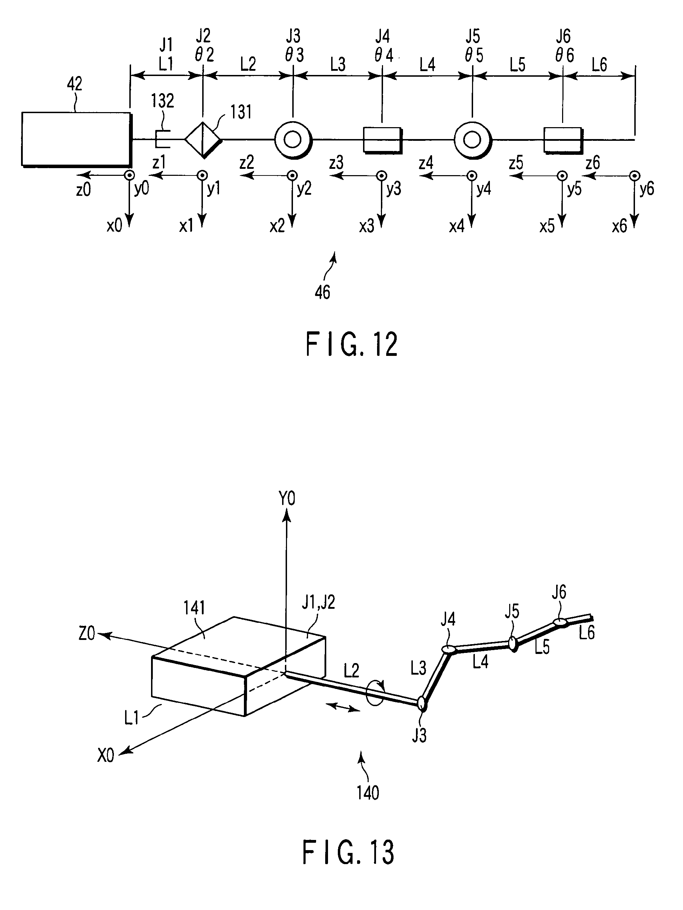

Next, another embodiment of the present invention will be explained, with reference to FIGS. 12 and 13. The overall configuration of an endoscope apparatus system in this embodiment is approximately the same as that of the above-described embodiment. However, a motor unit of a surgical instrument 40 is additionally provided with a mechanism 131 for rotating a bending portion 46 around the axis of an insertion portion 42 and a mechanism 132 for advancing the bending portion 46 in the axial direction of the insertion portion 42 in parallel therewith. Further, at least four joints are disposed in the bending portion 46. With this configuration, the position and the attitude of the extreme end portion 47 are arbitrarily controlled. Further, the movement of the surgical instrument 40 corresponds to that of a manipulation input unit 140. A joystick type manipulation input unit having an advancing, retreating, and rotating joint structure is used as the manipulation input unit 140.

A coordinate system is set as shown in FIG. 12. The coordinate system uses a base end portion 141 of the manipulation input unit 140 as a reference and corresponds to the surgical instrument 40. In the coordinate system, the joint J1 moves forward and rearward, the joint J2 rotates in an axial direction, the joints J3, J5 are bent about a Y-axis, and the joints J4, J6 are bent about an X-axis. The rotation angles of the joints J2 to J6 are shown by θ2 to θ6, respectively. The lengths of respective rods are shown by L1 to L5 and the length of an extreme end rod is shown by L6. Thus, conversion matrices in the respective joints J1, J2, J3, J4, J5, J6 are shown by Expression 1 from the kinematics of the manipulator (surgical instrument 40).

[Expression 1]

$$\text{Joint J1}: T_0^1 = \begin{pmatrix} 1 & 0 & 0 & 0 \\ 0 & 1 & 0 & 0 \\ 0 & 0 & 1 & -L_1 \\ 0 & 0 & 0 & 1 \end{pmatrix}$$

$$\text{Joint J2}: T_1^2 = \begin{pmatrix} \cos\theta_2 & -\sin\theta_2 & 0 & 0 \\ \sin\theta_2 & \cos\theta_2 & 0 & 0 \\ 0 & 0 & 1 & -L_2 \\ 0 & 0 & 0 & 1 \end{pmatrix}$$

$$\text{Joint J3}: T_2^3 = \begin{pmatrix} \cos\theta_3 & 0 & \sin\theta_3 & -L_3\sin\theta_3 \\ 0 & 1 & 0 & 0 \\ -\sin\theta_3 & 0 & \cos\theta_3 & -L_3\cos\theta_3 \\ 0 & 0 & 0 & 1 \end{pmatrix}$$

$$\text{Joint J4}: T_3^4 = \begin{pmatrix} 1 & 0 & 0 & 0 \\ 0 & \cos\theta_4 & -\sin\theta_4 & L_4\sin\theta_4 \\ 0 & \sin\theta_4 & \cos\theta_4 & -L_4\cos\theta_4 \\ 0 & 0 & 0 & 1 \end{pmatrix}$$

$$\text{Joint J5}: T_4^5 = \begin{pmatrix} \cos\theta_5 & 0 & \sin\theta_5 & -L_5\sin\theta_5 \\ 0 & 1 & 0 & 0 \\ -\sin\theta_5 & 0 & \cos\theta_5 & -L_5\cos\theta_5 \\ 0 & 0 & 0 & 1 \end{pmatrix}$$

$$\text{Joint J6}: T_5^6 = \begin{pmatrix} 1 & 0 & 0 & 0 \\ 0 & \cos\theta_6 & -\sin\theta_6 & L_6\sin\theta_6 \\ 0 & \sin\theta_6 & \cos\theta_6 & -L_6\cos\theta_6 \\ 0 & 0 & 0 & 1 \end{pmatrix}$$

Accordingly, a homogeneous conversion matrix is shown by Expression 2.

[Expression 2]

$$T_0^6 = T_0^1 T_1^2 T_2^3 T_3^4 T_4^5 T_5^6$$

$$= \begin{pmatrix} r_{11} & r_{12} & r_{13} & t_x \\ r_{21} & r_{22} & r_{23} & t_y \\ r_{31} & r_{32} & r_{33} & t_z \\ 0 & 0 & 0 & 1 \end{pmatrix}$$

Since the coordinate system uses the base end portion 141 as the reference, the position (x, y, z) and the attitude ($\theta$x, $\theta$y, $\theta$z) of the extreme end portion of the manipulation input unit 140 are determined by Expression 3.

[Expression 3]

$$\begin{pmatrix} x \\ y \\ z \end{pmatrix}^T = \begin{pmatrix} t_x \\ t_y \\ t_z \end{pmatrix}^T \qquad \begin{pmatrix} \theta_x \\ \theta_y \\ \theta_z \end{pmatrix}^T = \begin{pmatrix} \mathrm{asin}(r_{32}/\cos\theta_y) \\ \mathrm{asin}(-r_{31}) \\ \mathrm{asin}(r_{21}/\cos\theta_y) \end{pmatrix}^T$$

The configuration of the surgical instrument 40 is different from that of the manipulation input unit 140. Accordingly, to operate the surgical instrument 40 by operating the manipulation input unit 140, it is necessary to match the position and the attitude of the surgical instrument 40 with those of the manipulation input unit 140. For this purpose, the rotation angles and the amounts of parallel (forward and rearward) movement of the respective joints of the surgical instrument 40 must be determined.

As described above, the movement of the surgical instrument 40 corresponds to that of the manipulation input unit 140. Accordingly, the position and the attitude of the surgical instrument 40 are determined by those of the manipulation input unit 140. Assuming that the configuration of the surgical instrument 40 is known, the rotation angles and the amounts of parallel movement of the respective configurations of the surgical instrument 40 can be determined by inverse kinematics. Inverse kinematics is a method of estimating the specific values of the joints (angles and the like thereof) from the position/attitude information of the manipulator (surgical instrument 40) in a working space. The joint parameter $\Phi$ of the respective joints 1, 2, ..., n are shown by Expression 4.

[Expression 4]

$$\Phi = (\theta_1, \ \theta_2, \ \cdots, \ \theta_n)^T$$

The position and the attitude of the manipulator are shown by Expression 5.

[Expression 5]

$$E_p = (x_{Ep}, \ y_{Ep}, \ z_{Ep}, \ \mathrm{Roll}_{Ep}, \ \mathrm{Yaw}_{Ep}, \ \mathrm{Pitch}_{Ep})^T$$

Thus, the relation thereof is shown by Expression 6.

[Expression 6]

$$E_p = A(\Phi)$$

Here, the target P of the position and the attitude of the manipulator is shown by Expression 7.

[Expression 7]

$$P_p = (x_{Pp}, \ y_{Pp}, \ z_{Pp}, \ \mathrm{Roll}_{Pp}, \ \mathrm{Yaw}_{Pp}, \ \mathrm{Pitch}_{Pp})^T$$

To place the manipulator in a Pp state, $\Phi$ must be determined to satisfy Expression 8.

[Expression 8]

$$P_p = A(\Phi)$$

However, since these expressions are non-linear, ordinarily, Jacobian matrix J(Φ) is determined by subjecting Ep to partial differentiation by the factor of Φ to determine Φ.

[Expression 9]

$$J(\Phi) = \begin{pmatrix} dx_{ep}/d\theta_1 & dx_{ep}/d\theta_2 & \cdots & dx_{ep}/d\theta_n \\ dy_{ep}/d\theta_1 & dy_{ep}/d\theta_2 & \cdots & dy_{ep}/d\theta_n \\ dz_{ep}/d\theta_1 & dz_{ep}/d\theta_2 & \cdots & dz_{ep}/d\theta_n \\ dRoll_{ep}/d\theta_1 & dRoll_{ep}/d\theta_2 & \cdots & dRoll_{ep}/d\theta_n \\ dYaw_{ep}/d\theta_1 & dYaw_{ep}/d\theta_2 & \cdots & dYaw_{ep}/d\theta_n \\ dPitch_{ep}/d\theta_1 & dPitch_{ep}/d\theta_2 & \cdots & dPitch_{ep}/d\theta_n \end{pmatrix}$$

Expression 11 is determined from Expression 10.

[Expression 10]

$$\dot{\Phi} = J(\Phi)^{-1}\dot{E}_p$$

[Expression 11]

$$P_p = A(\Phi)$$

Then, Φ that satisfies Expression 11 is determined by a convergence calculation.

As a result, according to the embodiment, even when the configuration of the manipulation input unit 140 is different from that of the surgical instrument 40, the extreme end of the surgical instrument 40 can be moved to an arbitrary position and an arbitrary attitude from the position and the attitude of the manipulation input unit 140, and an affected area can be cut out and exfoliated more easily than ever before.

The present invention can be also applied to a bending portion of an endoscope. The present invention can be applied to, for example, the bending mechanism of the bending portion in the insertion portion of the endoscope according to the embodiment described above. Further, the surgical instrument as a target of the present invention also includes a surgical catheter.

<Additional statement>

According to the above explanation, there can be obtained multijointed medical equipment according to the following items or arbitrary combinations of the following items and the items according to the claims.

1. An endoscope surgical instrument including:

an endoscope to observe an affected area in a body cavity;
a surgical instrument to perform surgery on the affected area by passing through an insertion portion of the endoscope;
at least one bending means disposed in an extreme end of the surgical instrument;
manipulation means for moving the extreme end of the surgical instrument in a direction intended by an operator;

control means for controlling movement of the extreme end of the surgical instrument in response to manipulation of the manipulation means; and

means for operating the bending means of the surgical instrument in response to a control signal from the control means.

2. The endoscope surgical instrument according to item 1, wherein the surgical instrument includes a soft insertion portion and a surgical portion for cutting out and exfoliating an affected area of a living body.

3. The endoscope surgical instrument according to item 1, wherein power for operating the bending means is assembled in the vicinity of the extreme end of the surgical instrument.

4. The endoscope surgical instrument according to item 1, in which power for operating the bending means is disposed in a portion other than the vicinity of the extreme end of the surgical instrument and which includes transmission means for transmitting the power to the bending means.

5. The endoscope surgical instrument according to item 1, including means for moving the extreme end of the surgical instrument forward and rearward.

## Claims

1. A multijointed bending mechanism **characterized by** comprising:

   a first bending piece;
   a second bending piece connected to the first bending piece so as to be rotatable around a first rotation shaft;
   a third bending piece connected to the second bending piece so as to be rotatable around a second rotation shaft;
   at least two first wires connected to the first bending piece to rotate the first bending piece; and
   at least two second wires connected to the second bending piece to rotate the second bending piece,
   wherein the first wires are disposed inwards of the second wires with respect to a vertical direction of the first and second rotation shafts.

2. The multijointed bending mechanism according to claim 1, **characterized by** further comprising positioning/disposing means disposed in the second bending piece for executing positioning so that the first wires are disposed inwards of the second wires.

3. The multijointed bending mechanism according to claim 1, **characterized by** further comprising:

   a first elastic member connected to the second bending piece to guide the first wires; and
   a second elastic member connected to the third bending piece to guide the second wires.

4. The multijointed bending mechanism according to claim 3, **characterized by** further comprising positioning/disposing means disposed in the second bending piece for executing positioning so that the first wires are disposed inwards of the second wires.

5. A multijointed medical equipment comprising the multijointed bending mechanism according to claim 1.

6. A multijointed bending mechanism **characterized by** comprising:

   a first bending piece;
   a second bending piece connected to the first bending piece so as to be rotatable around a first rotation shaft;
   a third bending piece connected to the second bending piece so as to be rotatable around a second rotation shaft;
   at least two first wires connected to the first bending piece to rotate the first bending piece; and
   at least two third wires connected to the third bending piece to rotate the third bending piece,
   wherein the first wires are disposed inwards of the third wires with respect to a vertical direction of the first and second rotation shafts.

7. The multijointed bending mechanism according to claim 6, **characterized by** further comprising positioning/disposing means disposed in the third bending piece for executing positioning so that the first wires are disposed inwards of the third wires.

8. The multijointed bending mechanism according to claim 6, **characterized by** further comprising:

a first elastic member connected to the second bending piece to guide the first wires; and
a third elastic member connected to a fourth bending piece rotatably connected to the third bending piece to guide the third wires.

9. The multijointed bending mechanism according to claim 8, **characterized by** further comprising positioning/disposing means disposed in the third bending piece for executing positioning so that the first wires are disposed inwards of the third wires.

10. A multijointed medical equipment comprising the multijointed bending mechanism according to claim 6.

FIG.1

F I G. 2

EP 2 130 504 A1

F I G. 3

EP 2 130 504 A1

Up

Right

Left

Down

Center axis L of
bending portion

59a

63

70

57a

70

61    65

48    48a

70

B

A

A

B

48b

47

51

70    56b

52

62    53

70    58b

54    64    55

46

94

F I G. 4

F I G. 5A

F I G. 5B

A-A Cross section

# F I G. 6A

B-B Cross section

# F I G. 6B

C1-C1 Cross section

FIG. 6C

C2-C2 Cross section

FIG. 6D

D-D Cross section

# F I G. 6E

F I G. 7A

EP 2 130 504 A1

FIG. 7B

F I G. 8A

F I G. 8B

FIG. 9

FIG. 10A

A–A Cross section

B-B Cross section

F I G. 10B

C1-C1 Cross section

F I G. 10C

C2–C2 Cross section

F I G. 10D

D–D Cross section

F I G. 10E

52  71

66b
56b

66a
56a

76

A-A Cross section

FIG. 11A

57a  67a

53

72

66b
56b

77

66a
56a

67b  57b

B-B Cross section

FIG. 11B

C2-C2 Cross section

# F I G. 11C

D-D Cross section

# F I G. 11D

F I G. 12

F I G. 13

## INTERNATIONAL SEARCH REPORT

| | International application No. |
|---|---|
| | PCT/JP2007/074713 |

A. CLASSIFICATION OF SUBJECT MATTER
*A61B17/28(2006.01)i, A61B1/00(2006.01)i, A61B18/12(2006.01)i*

According to International Patent Classification (IPC) or to both national classification and IPC

B. FIELDS SEARCHED

Minimum documentation searched (classification system followed by classification symbols)
A61B17/28, A61B1/00, A61B18/12

Documentation searched other than minimum documentation to the extent that such documents are included in the fields searched
Jitsuyo Shinan Koho        1922-1996   Jitsuyo Shinan Toroku Koho   1996-2008
Kokai Jitsuyo Shinan Koho   1971-2008   Toroku Jitsuyo Shinan Koho   1994-2008

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 6-114000 A  (Olympus Optical Co., Ltd.), 26 April, 1994 (26.04.94), Par. Nos. [0048], [0049]; Fig. 23 (Family: none) | 1-10 |
| A | JP 2007-54400 A  (Olympus Medical Systems Corp.), 08 March, 2007 (08.03.07), Abstract; Fig. 3 (Family: none) | 1-10 |

☐ Further documents are listed in the continuation of Box C.     ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 12 February, 2008 (12.02.08) | 26 February, 2008 (26.02.08) |

| Name and mailing address of the ISA/ | Authorized officer |
|---|---|
| Japanese Patent Office | |
| Facsimile No. | Telephone No. |

Form PCT/ISA/210 (second sheet) (April 2007)

**REFERENCES CITED IN THE DESCRIPTION**

**Patent documents cited in the description**

- JP 2003126024 A
- JP 6105797 A